# EUROPEAN PATENT APPLICATION

(11) **EP 1 518 931 A1**
(43) Date of publication of application: **30.03.2005**
(21) Application number: 04027786.5
(22) Date of filing: 02.04.2001
(51) Int. Cl.: C12N 15/12, C07K 14/47, C12N 15/62, A61K 38/17, A61P 7/02

(54) **Use of P-selectin glycoprotein ligand-1 (PSGL-1) and fragments thereof for the inhibition of thrombosis**

(30) Priority: 31.03.2000 US 193787 P
(62) Divisional of application: 01920923.8
(71) Applicant: Genetics Institute, LLC, Cambridge, MA 02140 (US)
(72) Inventor: Eppihimer, Michael J., Belefonte, PA 16823 (US); Schaub, Robert G., Pelham, New Hampshire 03076 (US); Harris, Alan S., Andover, Massachusetts 01810 (US)
(74) Representative: VOSSIUS & PARTNER

(57) **Abstract**

The present invention relates to methods and compositions for the modulation of thrombosis, in a subject, by administering a P-selectin antagonist. The invention further provides methods for modulating leukocyte recruitment, cellular migration, leukocyte rolling velocity, intercellular adhesion, and cell adhesion to blood vessels in a subject by administering soluble P-selectin ligand or fragments thereof, an anti-P-selectin ligand antibody, or an anti-P-selectin antibody. The invention also provides methods for identifying compounds capable of modulating thrombosis.

SEQUENCE LISTING

<110> Genetics Institute

<120> Inhibition of Thrombosis by Treatment with
      P-Selectin Antagonists

<130> GFN-5398PC

<140>
<141>

<150> 60/193,787
<151> 2000-03-31

<160> 4

<170> PatentIn Ver. 2.0

<210> 1
<211> 1649
<212> DNA
<213> Homo sapiens

<400> 1
gccacttctt ctgggcccac gaggcagctg tcccatgctc tgctgagcac ggtggtgcca 60

tgcctctgca actcctcctg ttgctgatcc tactgggccc tggcaacagc ttgcagctgt 120

gggacacctg ggcagatgaa gccgagaaag ccttgggtcc cctgcttgcc cgggaccgga 180

gacaggccac cgaatatgag tacctagatt atgatttcct gccagaaacg gagcctccag 240

aaatgctgag gaacagcact gacaccactc ctctgactgg gcctggaacc cctgagtcta 300

ccactgtgga gcctgctgca aggcgttcta ctggcctgga tgcaggaggg gcagtcacag 360

agctgaccac ggagctggcc aacatgggga acctgtccac ggattcagca gctatggaga 420

tacagaccac tcaaccagca gccacggagg cacagaccac tccactggca gccacagagg 480

cacagacaac tcgactgacg gccacggagg cacagaccac tccactggca gccacagagg 540

cacagaccac tccaccagca gccacggaag cacagaccac tcaacccaca ggcctggagg 600

cacagaccac tgcaccagca gccatggagg cacagaccac tgcaccagca gccatggaag 660

cacagaccac tccaccagca gccatggagg cacagaccac tcaaaccaca gccatggagg 720

cacagaccac tgcaccagaa gccacggagg cacagaccac tcaacccaca gccacggagg 780

cacagaccac tccactggca gccatggagg ccctgtccac agaacccagt gccacagagg 840

ccctgtccat ggaacctact accaaaagag gtctgttcat acccttttct gtgtcctctg 900

ttactcacaa gggcattccc atggcagcca gcaatttgtc cgtcaactac ccagtggggg 960

ccccagacca catctctgtg aagcagtgcc tgctggccat cctaatcttg gcgctggtgg 1020

ccactatctt cttcgtgtgc actgtggtgc tggcggtccg cctctcccgc aagggccaca 1080

tgtaccccgt gcgtaattac tcccccaccg agatggtctg catctcatcc ctgttgcctg 1140

atgggggtga ggggccctct gccacagcca atgggggcct gtccaaggcc aagagcccgg 1200

gcctgacgcc agagcccagg gaggaccgtg agggggatga cctcaccctg cacagcttcc 1260

tcccttagct cactctgcca tctgttttgg caagacccca cctccacggg ctctcctggg 1320

ccacccctga gtgcccagac cccaatccac agctctgggc ttcctcggag acccctgggg 1380

atggggatct tcagggaagg aactctggcc acccaaacag gacaagagca gcctggggcc 1440

aagcagacgg gcaagtggag ccacctcttt cctccctccg cggatgaagc ccagccacat 1500

ttcagccgag gtccaaggca ggaggccatt tacttgagac agattctctc ctttttcctg 1560

tcccccatct tctctgggtc cctctaacat ctcccatggc tctccccgct tctcctggtc 1620

actggagtct cctccccatg tacccaagg 1649

<210> 2
<211> 402
<212> PRT
<213> Homo sapiens

<400> 2
Met Pro Leu Gln Leu Leu Leu Leu Leu Ile Leu Leu Gly Pro Gly Asn
1               5                   10                  15

Ser Leu Gln Leu Trp Asp Thr Trp Ala Asp Glu Ala Glu Lys Ala Leu
            20                  25                  30

Gly Pro Leu Leu Ala Arg Asp Arg Arg Gln Ala Thr Glu Tyr Glu Tyr
        35                  40                  45

Leu Asp Tyr Asp Phe Leu Pro Glu Thr Glu Pro Pro Glu Met Leu Arg
    50                  55                  60

Asn Ser Thr Asp Thr Thr Pro Leu Thr Gly Pro Gly Thr Pro Glu Ser
65                  70                  75                  80

Thr Thr Val Glu Pro Ala Ala Arg Arg Ser Thr Gly Leu Asp Ala Gly
                85                  90                  95

Gly Ala Val Thr Glu Leu Thr Thr Glu Leu Ala Asn Met Gly Asn Leu
            100                 105                 110

Ser Thr Asp Ser Ala Ala Met Glu Ile Gln Thr Thr Gln Pro Ala Ala
        115                 120                 125

Thr Glu Ala Gln Thr Thr Pro Leu Ala Ala Thr Glu Ala Gln Thr Thr
    130                 135                 140

Arg Leu Thr Ala Thr Glu Ala Gln Thr Thr Pro Leu Ala Ala Thr Glu
145                 150                 155                 160

Ala Gln Thr Thr Pro Pro Ala Ala Thr Glu Ala Gln Thr Thr Gln Pro
                165                 170                 175

Thr Gly Leu Glu Ala Gln Thr Thr Ala Pro Ala Ala Met Glu Ala Gln

180                 185                 190

Thr Thr Ala Pro Ala Ala Met Glu Ala Gln Thr Thr Pro Pro Ala Ala
        195                 200                 205

Met Glu Ala Gln Thr Thr Gln Thr Thr Ala Met Glu Ala Gln Thr Thr
    210                 215                 220

Ala Pro Glu Ala Thr Glu Ala Gln Thr Thr Gln Pro Thr Ala Thr Glu
225                 230                 235                 240

Ala Gln Thr Thr Pro Leu Ala Ala Met Glu Ala Leu Ser Thr Glu Pro
                245                 250                 255

Ser Ala Thr Glu Ala Leu Ser Met Glu Pro Thr Thr Lys Arg Gly Leu
            260                 265                 270

Phe Ile Pro Phe Ser Val Ser Ser Val Thr His Lys Gly Ile Pro Met
        275                 280                 285

Ala Ala Ser Asn Leu Ser Val Asn Tyr Pro Val Gly Ala Pro Asp His
    290                 295                 300

Ile Ser Val Lys Gln Cys Leu Leu Ala Ile Leu Ile Leu Ala Leu Val
305                 310                 315                 320

Ala Thr Ile Phe Phe Val Cys Thr Val Val Leu Ala Val Arg Leu Ser
                325                 330                 335

Arg Lys Gly His Met Tyr Pro Val Arg Asn Tyr Ser Pro Thr Glu Met
            340                 345                 350

Val Cys Ile Ser Ser Leu Leu Pro Asp Gly Gly Glu Gly Pro Ser Ala
        355                 360                 365

Thr Ala Asn Gly Gly Leu Ser Lys Ala Lys Ser Pro Gly Leu Thr Pro
    370                 375                 380

Glu Pro Arg Glu Asp Arg Glu Gly Asp Asp Leu Thr Leu His Ser Phe
385                 390                 395                 400

Leu Pro


<210> 3
<211> 942
<212> DNA
<213> Homo sapiens

<220>
<221> CDS
<222> (1)..(939)

<400> 3
atg cct ctg caa ctc ctc ctg ttg ctg atc cta ctg ggc cct ggc aac      48
Met Pro Leu Gln Leu Leu Leu Leu Leu Ile Leu Leu Gly Pro Gly Asn
1               5                   10                  15

agc ttg cag ctg tgg gac acc tgg gca gat gaa gcc gag aaa gcc ttg      96
Ser Leu Gln Leu Trp Asp Thr Trp Ala Asp Glu Ala Glu Lys Ala Leu

20                  25                  30

ggt ccc ctg ctt gcc cgg gac cgg aga cag gcc acc gaa tat gag tac     144
Gly Pro Leu Leu Ala Arg Asp Arg Arg Gln Ala Thr Glu Tyr Glu Tyr
        35                  40                  45

cta gat tat gat ttc ctg cca gaa acg gag cct cca gaa atg ctg agg     192
Leu Asp Tyr Asp Phe Leu Pro Glu Thr Glu Pro Pro Glu Met Leu Arg
    50                  55                  60

aac agc act gac acc act cct ctg act ggg cct gga acc cct gag tct     240
Asn Ser Thr Asp Thr Thr Pro Leu Thr Gly Pro Gly Thr Pro Glu Ser
65                  70                  75                  80

acc act gtg gag cct gct gcg cgg ccg cac aca tgc cca ccg tgc cca     288
Thr Thr Val Glu Pro Ala Ala Arg Pro His Thr Cys Pro Pro Cys Pro
                85                  90                  95

gca cct gaa gcc ctg ggg gca ccg tca gtc ttc ctc ttc ccc cca aaa     336
Ala Pro Glu Ala Leu Gly Ala Pro Ser Val Phe Leu Phe Pro Pro Lys
            100                 105                 110

ccc aag gac acc ctc atg atc tcc cgg acc cct gag gtc aca tgc gtg     384
Pro Lys Asp Thr Leu Met Ile Ser Arg Thr Pro Glu Val Thr Cys Val
        115                 120                 125

gtg gtg gac gtg agc cac gaa gac cct gag gtc aag ttc aac tgg tac     432
Val Val Asp Val Ser His Glu Asp Pro Glu Val Lys Phe Asn Trp Tyr
    130                 135                 140

gtg gac ggc gtg gag gtg cat aat gcc aag aca aag ccg cgg gag gag     480
Val Asp Gly Val Glu Val His Asn Ala Lys Thr Lys Pro Arg Glu Glu
145                 150                 155                 160

cag tac aac agc acg tac cgt gtg gtc agc gtc ctc acc gtc ctg cac     528
Gln Tyr Asn Ser Thr Tyr Arg Val Val Ser Val Leu Thr Val Leu His
                165                 170                 175

cag gac tgg ctg aat ggc aag gag tac aag tgc aag gtc tcc aac aaa     576
Gln Asp Trp Leu Asn Gly Lys Glu Tyr Lys Cys Lys Val Ser Asn Lys
            180                 185                 190

gcc ctc cca gtc ccc atc gag aaa acc atc tcc aaa gcc aaa ggg cag     624
Ala Leu Pro Val Pro Ile Glu Lys Thr Ile Ser Lys Ala Lys Gly Gln
        195                 200                 205

ccc cga gaa cca cag gtg tac acc ctg ccc cca tcc cgg gag gag atg     672
Pro Arg Glu Pro Gln Val Tyr Thr Leu Pro Pro Ser Arg Glu Glu Met
    210                 215                 220

acc aag aac cag gtc agc ctg acc tgc ctg gtc aaa ggc ttc tat ccc     720
Thr Lys Asn Gln Val Ser Leu Thr Cys Leu Val Lys Gly Phe Tyr Pro
225                 230                 235                 240

agc gac atc gcc gtg gag tgg gag agc aat ggg cag ccg gag aac aac     768
Ser Asp Ile Ala Val Glu Trp Glu Ser Asn Gly Gln Pro Glu Asn Asn
                245                 250                 255

tac aag acc acg cct ccc gtg ctg gac tcc gac ggc tcc ttc ttc ctc     816
Tyr Lys Thr Thr Pro Pro Val Leu Asp Ser Asp Gly Ser Phe Phe Leu
            260                 265                 270

tat agc aag ctc acc gtg gac aag agc agg tgg cag cag ggg aac gtc     864
Tyr Ser Lys Leu Thr Val Asp Lys Ser Arg Trp Gln Gln Gly Asn Val
        275                 280                 285

ttc tca tgc tcc gtg atg cat gag gct ctg cac aac cac tac acg cag     912
Phe Ser Cys Ser Val Met His Glu Ala Leu His Asn His Tyr Thr Gln
    290                 295                 300

aag agc ctc tcc ctg tcc ccg ggt aaa tga                             942
Lys Ser Leu Ser Leu Ser Pro Gly Lys
305                 310

<210> 4
<211> 313
<212> PRT
<213> Homo sapiens

<400> 4
Met Pro Leu Gln Leu Leu Leu Leu Leu Ile Leu Leu Gly Pro Gly Asn
1               5                   10                  15

Ser Leu Gln Leu Trp Asp Thr Trp Ala Asp Glu Ala Glu Lys Ala Leu
            20                  25                  30

Gly Pro Leu Leu Ala Arg Asp Arg Arg Gln Ala Thr Glu Tyr Glu Tyr
        35                  40                  45

Leu Asp Tyr Asp Phe Leu Pro Glu Thr Glu Pro Pro Glu Met Leu Arg
    50                  55                  60

Asn Ser Thr Asp Thr Thr Pro Leu Thr Gly Pro Gly Thr Pro Glu Ser
65                  70                  75                  80

Thr Thr Val Glu Pro Ala Ala Arg Pro His Thr Cys Pro Pro Cys Pro
                85                  90                  95

Ala Pro Glu Ala Leu Gly Ala Pro Ser Val Phe Leu Phe Pro Pro Lys
            100                 105                 110

Pro Lys Asp Thr Leu Met Ile Ser Arg Thr Pro Glu Val Thr Cys Val
        115                 120                 125

Val Val Asp Val Ser His Glu Asp Pro Glu Val Lys Phe Asn Trp Tyr
    130                 135                 140

Val Asp Gly Val Glu Val His Asn Ala Lys Thr Lys Pro Arg Glu Glu
145                 150                 155                 160

Gln Tyr Asn Ser Thr Tyr Arg Val Val Ser Val Leu Thr Val Leu His
                165                 170                 175

Gln Asp Trp Leu Asn Gly Lys Glu Tyr Lys Cys Lys Val Ser Asn Lys
            180                 185                 190

Ala Leu Pro Val Pro Ile Glu Lys Thr Ile Ser Lys Ala Lys Gly Gln
        195                 200                 205

Pro Arg Glu Pro Gln Val Tyr Thr Leu Pro Pro Ser Arg Glu Glu Met
    210                 215                 220

Thr Lys Asn Gln Val Ser Leu Thr Cys Leu Val Lys Gly Phe Tyr Pro
225                 230                 235                 240

Ser Asp Ile Ala Val Glu Trp Glu Ser Asn Gly Gln Pro Glu Asn Asn
                245                 250                 255

Tyr Lys Thr Thr Pro Pro Val Leu Asp Ser Asp Gly Ser Phe Phe Leu
            260                 265                 270

Tyr Ser Lys Leu Thr Val Asp Lys Ser Arg Trp Gln Gln Gly Asn Val
        275                 280                 285

Phe Ser Cys Ser Val Met His Glu Ala Leu His Asn His Tyr Thr Gln
    290                 295                 300

Lys Ser Leu Ser Leu Ser Pro Gly Lys
305                 310

## Description

### Related Applications

This application claims priority to U.S. Provisional Patent Application. No. 60/193,787 filed on March 31,2000, incorporated herein in its entirety by reference.

### Background of the Invention

Thrombosis is the formation and development of a blood clot, or thrombus, within a blood vessel which can either partially or completely block the flow of blood in the vein. Deep vein thrombosis (DVT) is the formation of a thrombus within a deep vein. The thrombus can block a vessel and stop blood supply to an organ or other body part. If detached, the thrombus can become an embolus and occlude a vessel distant from the original site or lead to pulmonary emboli. DVT is common in patients who are immobilized for relatively long periods of time as a result of a medical or surgical illness, or patients with multiple trauma or malignant diseases. DVT may also develop in otherwise healthy persons, after prolonged sitting or immobilization. Leukocyte adhesion, transendothelial migration, and stasis are important components in the pathogenesis of DVT. (Eppihimer and Schaub, (2000) *Arteriosclerosis Thromb Vasc Biol* 20:2483).

Thrombosis is a serious condition, which can cause tissue damage, and if untreated, eventually death. Thrombosis reflects, in part, an imbalance between procoagulant and anticoagulant mechanisms (Gross and Aird (2000) *Semin Thromb Hemostat* 26:463) and thrombotic formation is dependent upon platelet and leukocyte aggregation. The interaction of platelets with other platelets and with the endothelial surface of injured blood vessels is a major factor in thrombotic development. Physical injury of an arterial wall may result from vascular intervention procedures such as percutaneous transluminal coronary angioplasty (PTCA) or coronary bypass surgery, leading to the formation of thrombotic reocculsion. Or, thrombosis may result from the progression of a natural disease, such as atherosclerosis. Thrombi that form on atherosclerotic lesions in coronaries are responsible for myocardial ischemia and progression of atherosclerosis (Rauch, *et al*. (2001) Annals *of Internal Medicine* 134(3):224). Moreover, one of the basic pathophysiological processes underlying the major complications of hypertension (*i.e.*, heart attack and stroke) is thrombogenesis (Lip (2000) *J Hum Hypertension* 14:687. Soluble P-selectin has been identified as a direct inducer of pro-coagulative activity associated with vascular and thrombotic diseases (Andre, *et al*. (2000) *Proc Natl Acad Sci USA 97:13835):* Selectins, *e.g.,* P-selectin, E-selectin, and L-selectin, are believed to mediate intercellular adhesion through specific interactions with ligands present on the surface of target cells, *e.g.*, platelets and leukocytes. Generally the ligands of selectins are comprised at least in part of a carbohydrate moiety (*e.g.*; sialyl Lewis^{x} (sLe^{x}) and sialyl Lewis^{a} (sLe^{a})). P-selectin binds to carbohydrates containing the non-sialated form of the Lewis^{x} blood group antigen and with higher affinity to sialyl Lewis^{x}. P-selectin Glycoprotein Ligand-1 (PSGL-1), a high-affinity P-selectin ligand which may also bind to E-selectin and L-selectin, is expressed by leukocytes and mediates cell adhesion between leukocytes, platelets, and endothelial cell-types (U.S. Patent Number 5,843,707 and U.S. Patent Number 5,827,817).

### Summary of the Invention

The present invention provides methods and compositions for the modulation, (*e.g*., prevention, inhibition, or treatment) of thrombosis. The present invention is based, at least in part, on the discovery that P-selectin antagonism by P-selectin antagonists, including P-selectin ligand molecules (or a fragment thereof having P-selectin ligand activity, *e*.*g*., soluble PSGL-1, or a soluble recombinant PSGL fusion protein, *e.g.*, dimeric PSGL-1), anti-P-selectin antibodies, and anti-P-selectin ligand antibodies inhibit cellular adhesion, *(e.g.,* cell to cell adhesion, *e*.*g*., leukocyte-endothelial or leukocyte-platelet adhesion) and cell (*e.g*., platelet or leukocyte) adhesion to blood vessels, modulate *(e.g,* increase) movement of cells *(e.g.,* leukocytes or platelets) relative to blood vessels, and increase leukocite rolling velocity, thereby inhibiting formation of thrombosis. The P-selectin ligand molecules of the invention are referred to herein as P-Selectin Glycoprotein Ligand-1 (PSGL-1) molecules.

In one aspect, the invention provides a method for modulating (*e*.*g*., preventing, inhibiting, or treating) thrombosis in a subject by administering a composition which includes an effective amount of a P-selectin antagonist. In one embodiment, the P-selectin antagonist is a P-selectin ligand protein. In another embodiment, the P-selectin ligand protein is a human P-selectin ligand protein. In a preferred embodiment, the P-selectin antagonist is a soluble P-selectin ligand protein, or a fragment thereof having P-selectin ligand activity, *e.g*., soluble PSGL-1, or a soluble recombinant PSGL fusion protein, *e.g*., a dimeric PSGL-1 or recombinant PSGL-Ig.. In another embodiment, the P-selectin antagonist is an anti-P-selectin antibody or biologically active fragment thereof, or an auti-P-selectin ligand antibody or biologically active fragment thereof. In a yet another embodiment, the composition further includes a pharmaceutically acceptable carrier.

In one embodiment the subject is a mammal, *e.g.,* a human. In another embodiment, the methods of the invention includes the administration of a soluble P-selectin ligand protein including at least a portion of an extracellular domain of a P-selectin ligand protein, for example, amino acids 42 to 60, 42 to 88, 42 to 118, 42 to 189, or 42 to 310, of the amino acid sequence set forth in SEQ ID NO:2. In another embodiment, the protein is a soluble P-selectin ligand protein including at least an extracellular domain of a P-selectin ligand protein set forth in SEQ ID NO:2. In a further embodiment, the invention provides that the soluble protein further including an Fc portion of an immunoglobulin, *e*.*g*., human IgG. In a related embodiment, the soluble protein is a soluble P-selectin ligand protein including the amino acid sequence from amino acid 42 to amino acid 60 of SEQ ID NO:2 fused at its C-terminus to the Fc portion of an immunoglobulin. In a related embodiment, the soluble protein is a soluble P-selectin ligand protein including the amino acid sequence from amino acid 42 to amino acid 88 of SEQ ID NO:2 fused at its C-terminus to the Fc portion of an immunoglobulin. In a further embodiment, the Fc portion of an immunoglobulin is fused to the P-selectin ligand protein through a linking sequence.

Another aspect of the invention provides a method for modulating (*e*.*g*., increasing) cell (*e*.*g*., leukocyte or platelet) movement relative to blood vessels or increasing leukocyte rolling velocity in a subject by administering a P-selectin antagonist, *e*.*g*., soluble PSGL-1 or a soluble recombinant PSGL fusion protein, *e.g.,* dimeric PSGL-1 or recombinant PSGL-Ig, an anti-P-selectin ligand antibody or biologically active fragment thereof, or an anti-P-selectin antibody or biologically active fragment thereof. Yet another aspect of the invention provides a method for inhibiting cell to cell adhesion in a subject by administering a P-selectin antagonist, e.g., soluble PSGL-1 or a soluble recombinant PSGL fusion protein, e.g. dimeric PSGL-1 or recombinant PSGL-Ig, an anti-P-selectin ligand antibody or biologically active fragment thereof, or an anti-P-selectin antibody or biologically active fragment thereof. In one embodiment, the adhesive cells are selected from the group consisting of leukocytes, platelets, and endothelial cells. A further aspect of the invention provides a method for inhibiting cell adhesion to blood vessels in a subject by administering a P-selectin antagonist, *e.g.*, soluble PSGL-1 or a soluble recombinant PSGL fusion protein, *e.g.*,dimeric PSGL-1 or recombinant PSGL-Ig, an anti-P-selectin ligand antibody or biologically active fragment thereof, or an anti-P-selectin antibody or biologically active fragment thereof, thereby inhibiting cell adhesion to blood vessels. In one embodiment, the adhesive cells are selected from the group consisting of leukocytes, platelets and endothelial cells.

In yet another aspect, the invention provides a method for identifying a compound, capable of modulating thrombosis in which the ability of the compound to modulate PSGL-1 polypeptide activity is assayed. In one embodiment, the ability of the compound to modulate PSGL-1 polypeptide activity is determined by detecting a decrease in cellular adhesion, *e.g.*, intercellular adhesion (*e.g.*, leukocyte-endothelial cell or leukocyte-platelet adhesion) and cell (*e.g.*, platelet or leukocyte) adhesion to blood vessels. In another embodiment, the ability of the compound to modulate PSGL-1 polypeptide activity is determined by detecting an increase in cell movement relative to blood vessels.

Other features and advantages of the invention will be apparent from the following detailed description and claims.

### Brief Description of the Drawings

*Figure 1* is a schematic representation of the structure of PSGL-1 molecules.

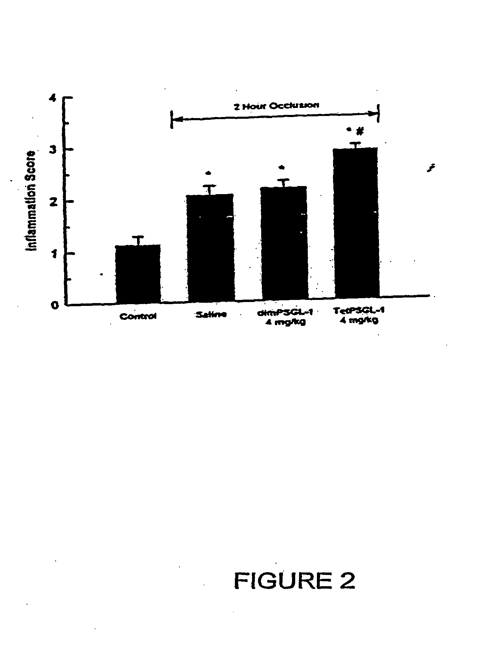

*Figure 2* is a graph depicting the effect of dimPSGL-1 (dimeric PSGL-1). and tetPSGL-1 (tetrameric PSGL-1) on venous wall inflammation following venous occlusion.
*Figure 3* is a graph depicting the effect of dimPSGL-1 and tetPSGL-1 on leukocyte rolling under basal conditions.
*Figure 4* is a graph depicting the effect of dimPSGL-1 and tetPSGL-1 on leukocyte rolling allowing exposure to LTC₄.
*Figure 5* is a graph depicting the effect of dimPSGL-1 and tetPSGL-1 on leukocyte adhesion following exposure to LTC₄.

### Detailed Description of the Invention

The present invention provides methods and compositions for the modulation, *e.g.,* prevention, inhibition, or treatment, of thrombosis, *in vivo*, by administration of a P-selectin antagonist *e*.*g*., a soluble P-selectin ligand protein, or a fragment thereof having P-selectin ligand activity, *e*.*g*., soluble PSGL-1, or a soluble recombinant PSGL fusion protein, *e*.*g*., dimeric PSGL-1, an anti-P-selectin ligand antibody or biologically active fragments thereof, or an anti-P-selectin antibody or biologically active fragments thereof. The P-selectin ligand proteins used in the methods of the invention are referred to herein as P-Selectin Glytoprotein Ligand-1- (PSGL-1) molecules.

The present invention is based, at least in part, on the discovery that soluble P-selectin ligand (*e*.*g*., dimeric PSGL-1) molecules inhibit the effect of thrombus-inducing agents, such as, for example, leukotriene C₄ (LTC₄), inhibit LTC₄-induced reduced leukocyte rolling velocity, attenuate subsequent cellular adhesion, and inhibit thrombosis in an animal model of deep vein thrombosis (DVT) (see Example 2).

As used herein, a thrombus-inducing agent includes any agent which induces formation of a blood clot, or thrombus, within a blood vessel. A thrombus-inducing agent also includes an agent which induces increased cell adhesion, decreased cell migration or movement relative to blood vessels, or decreased leukocyte rolling, thereby inducing thrombus formation within a blood vessel. Examples of thrombus- inducing agents include, but are not limited to, pharmaceutical compositions, naturally occurring agents produced within the body or administered to a subject, and agents which cause damage to blood vessels, *e.g.*, surgical procedure.

Cell to cell adhesion (*e*.*g*., leukocyte-endothelial cell or leukocyte-platelet adhesion), stasis, reduced movement of cells (*e.g.*, leukocytes and platelets) in relation to blood vessels, and reduced leukocyte rolling velocity contribute to thrombosis or the formation of a thrombus. Furthermore, leukocytes adhere to damaged blood vessels (*e*.*g*., endothelial cells) after vascular injury through binding of P-selectin and E-selectin with a P-selectin ligand; *e.g.,* PSGL-1, which is expressed on leukocytes, resulting in the formation of a thrombus. In one aspect of the invention, antagonism of P-selectin by, *e.g.,* a soluble P-selectin ligand protein, or a Bragment thereof having P-selectin ligand activity, *e.g.,* soluble PSGL-1, or a soluble recombinant PSGL fusion protein, *e*.*g*.,dimeric PSGL-1, an anti-P-selectin ligand antibody, or an anti-P-selectin antibody inhibits leukocyte adhesion to damaged arterial segments, modulates cellular adhesion, *e.g.,* intercellular adhesion *(e.g.,* leukocyte-endothelial cell or leukocyte platelet adhesion) and cell (*e.g.*, platelet or leukocyte) adhesion to blood vessels, modulates leukocyte recruitment to platelets and endothelial cells, modulates cell *(e.g.,* leukocyte or platelet) migration, modulates movement of cells relative to blood vessels, and modulates, *e.g.,* increases, leukocyte rolling velocity, thereby modulating (*e.g.*, preventing, inhibiting, or treating) thrombosis.

As used herein, a "P-selectin antagonist" includes any agent which is capable of antagonizing P-selectin and/or E-selectin, *e.g.*, by inhibiting interaction between P-selectin or E-selectin and a P-selectin ligand protein, e.g., by inhibiting interaction of P-selectin or E-selectin expressing endothelial cells and activated platelets with PSGL expressing leukocytes. For example, P-selectin antagonists include P-selectin ligand molecules, or a fragment thereof having P-selectin ligand activity, *e.g.,* soluble PSGL-1, or a soluble recombinant PSGL fusion protein, *e.g.*, recombinant PSGL-Ig, as well as small molecules, anti-P-selectin antibodies, and anti-P-selectin ligand antibodies. In a preferred embodiment; the P-selectin ligand is soluble. P-selectin antagonists for use in inhibition of thrombosis, as described herein, preferably do not include tetrameric P-selectin ligand molecules (tetPSGL-1 molecules), as described herein, or other antagonists which increase cross-linkage between leukocytes *in vitro,* thereby increasing thrombus formation.

As used interchangeably herein, "P-selectin ligand activity," "PSGL-1 activity," "biological activity of PSGL-1" or "functional activity of PSGL-1," includes. an activity exerted by a-PSGL-1 protein; polypeptide or nucleic: acid molecule on a PSGL-1 responsive cell, *e.g.*, platelet, leukocyte, or endothelial cell, as determined *in vivo,* or *in vitro,* according to standard techniques. PSGL-1 activity can be a direct activity, such as an association with a PSGL-1-target molecule *e.g.*, P-selectin or E-selectin. As used herein, a "substrate'' or "target molecule" or "binding partner" is a molecule, *e.g.* P-selectin or E-selectin, with which a PSGL-1 protein binds or interacts in nature, such that PSGL-1-mediated function, *e.g.*, modulation of cell migration or adhesion, is achieved. A PSGL-1 target molecule can be a non-PSGL-1 molecule or a PSGL-1 protein or polypeptide. Examples of such target molecules include proteins in the same signaling path as the PSGL-1 protein, *e.g.*, proteins which may function upstream (including both stimulators and inhibitors of activity) or downstream of the PSGL-1 protein in a pathway involving regulation of P-selectin binding. Alternatively, a PSGL-1 activity is an indirect activity, such as a cellular signaling activity mediated by interaction of the PSGL-1 protein with a PSGL-1 target molecule, *e.g*., P-selectin or E-selectin. The biological activities of PSGL-1 are described herein, and include, for example, one or more of the following activities: 1) binding to or interacting with P-selectin or E-selectin; 2) modulating P-selectin or E-selectin binding; 3) modulating cellular adhesion, *e.g.*, intercellular adhesion (*e.g.*, leukocyte-endothelial cell or leukocyte-platelet adhesion) and cell (*e.g.*, platelet or leukocyte) adhesion to blood vessels; 4) modulating leukocyte recruitment to platelets and endothelial cells; 5) modulating cell (*e.g*., leukocyte or platelet) migration; 6) modulating movement of cells relative to blood vessels; 7) modulating, *e.g.*, increasing, leukocyte rolling velocity; and 8) modulating, e.g., inhibiting, treating, or preventing, thrombosis.

As used herein, "thrombosis" includes the formation or development of one or more blood clots or thrombus within a blood vessel. As used herein, thrombosis also includes "deep vein thrombosis" (DVT), which is the formation of a thrombus within a deep vein, such as in the legs. Once formed, the thrombus may either partially or completely block the flow of blood in the blood vessel. The formation of a thrombus is caused, at least in part, by cell to cell adhesion (*e.g.*, leukocyte-endothelial cell or leukocyte-platelet adhesion), cell adhesion to blood vessels (*e.g.*, injured blood vessels), reduced movement or migration of cells (*e.g*., leukocytes or platelets) in relation to blood vessels, and/or reduced leukocyte rolling velocity.

A subject who may be at risk for thrombosis is one who suffers from a cardiovascular disease or disorder, *e.g.*, atherosclerosis or hypertension. A subject who may also be at risk for thrombosis is one who has undergone cardiovascular or general vascular procedures or intervention such as angioplasty of any vessel, *e.g*., carotid, femoral, coronary, etc.; surgical revascularization, *e.g.*, balloon angioplasty, laser angioplasty, percutaneous transluminal coronary angioplasty (PTCA), coronary artery bypass grafting, rotational atherectomy or coronary artery stents, or other intervention, surgical or non-surgical, which may cause vascular injury. A subject may also be at risk for thrombosis following any surgical procedure. Furthermore, a subject may be at risk for thrombosis, *e.g.,* DVT, if the subject is immobilized for prolonged periods of time, such as, for example, a patient during hospitalization. Healthy individuals may also be at risk due to long periods of immobilization, such as, for example, sitting during long trips. Administration of a P-selectin antagonist to modulate thrombosis may be prior to injury, during an intervention procedure, or after the injury or intervention has occurred. In a preferred embodiment, administration of the P-selectin antagonist is prior to surgical intervention, injury, or the onset of thrombus formation.

The PSGL-1 molecules used in the methods of the invention are described in U.S. Patent Number 5,827,817, the contents of which are incorporated herein by reference.

The PSGL-1 molecule used in the methods of the invention is a glycoprotein which may contain one or more of the following terminal carbohydrates: 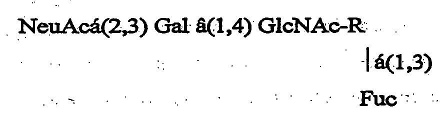 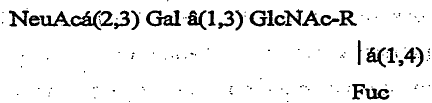 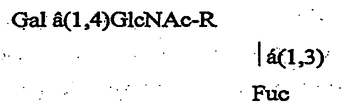 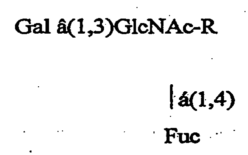 where R= the remainder of the carbohydrate chain, which is covalently attached either directly to the P-selectin ligand protein or to a lipid moiety which is covalently attached to the P-selectin ligand protein. The P-selectin ligand glycoprotein used in the methods of the invention may additionally be sulfated or otherwise post-translationally modified. As expressed in COS and CHO cells, full length P-selectin ligand protein (amino acids 1 to 402 of SEQ ID NO:2) or mature P-selectin ligand protein (amino acids 42 to 402 of SEQ ID NO:2) is a homodimeric or bivalent protein having an apparent molecular weight of 220 kD as shown by non-reducing SDS-polyacrylamide gel electrophoresis.

PGSL-1 is a glycoprotein which acts as a ligand for P-selectin and E-selectin on endothelial cells and platelets. The DNA sequence of PSGL-1 is set forth in SEQ ID NO:1. The complete amino acid sequence of the PSGL-1, *i.e.,* the mature peptide plus the leader sequence, is characterized by the amino acid sequence set forth in SEQ ID NO:2, from amino acid 1 to amino acid 402. The mature PSGL-1 protein is characterized by the amino acid sequence set forth in SEQ IQ NO:2 from amino acid 42 to amino acid 402.

As used herein, a "soluble PSGL-1 protein," or a "soluble P-selectin ligand protein," refers to a soluble P-selectin ligand glycoprotein, *e.g.*, soluble PSGL-1, or a fragment thereof having a P-selectin ligand activity, which includes a carbohydrate comprising sLe^{x}. Soluble P-selectin ligand proteins used in the methods of the invention preferably include at least an extracellular domain of PSGL-1, from about amino acid 18 to about amino acid 310 of SEQ ID NO:2, or a biologically active fragment thereof. Other soluble forms of the P-selectin ligand molecules are characterized by the amino acid sequence set forth in SEQ ID NO:2 from, *e.g.*, amino acids 42 to 310, or a biologically active fragment thereof. Biologically active fragments of the extracellular domain of the PSGL-1 include, for example, amino acids 42 to 60, 42 to 88, 42 to 118, and 42 to 189, of the amino acid sequence set forth in SEQ ID NO:2. Soluble PSGL-1 proteins used in the methods of the invention are preferably monomeric or dimeric PSGL-1 proteins.

In one embodiment of the methods of the invention, soluble forms of the P-selectin ligand molecules of the methods of the invention may be fused through "linker" sequences to the Fc portion of an immunoglobulin, *e.g.,* an IgG molecule, to form fusion proteins. Other immunoglobulin isotypes may also be used to generate such fusion proteins, provided that the resulting fusion protein is either monomeric or dimeric.

In another embodiment of the invention, the soluble P-selectin ligand protein is a chimeric molecule which is comprised of the extracellular domain of a PSGL-1 protein molecule, a carbohydrate comprising sLe^{x}, and is fused through linker sequences to the Fc portion of human IgG.

Monomeric forms of PSGL-1 may be produced, for example, by altering the amino acid sequence of PSGL-1 such that the cysteine at position 310 of SEQ ID NO:2 is replaced with a serine or an alanine, or by other methods known in the art.

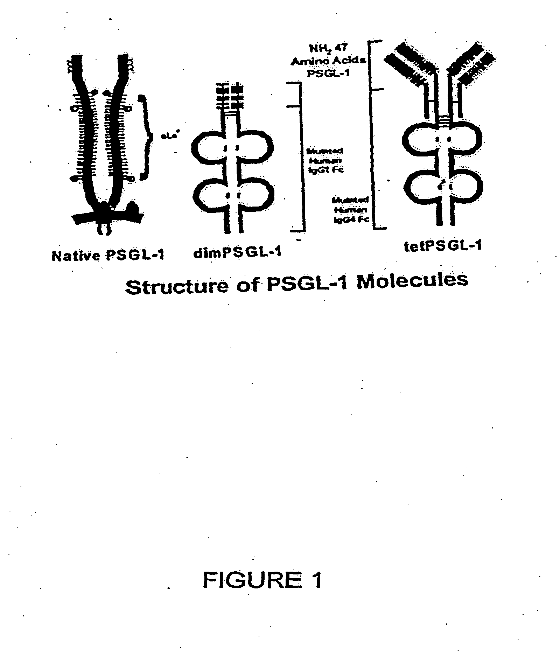

In a preferred embodiment, a dimeric PSGL-1 (dimPSGL-1) fusion protein is produced by truncating the NH₂ 47 amino acids of native PSGL-1, thereby maintaining a high affinity for P-selectin, but reducing binding to L-selectin and E-selectin (see Figure 1). The NH₂ 47 amino acids of PSGL-1 were linked to a Fc portion of human immunoglobulin-1 (IgG₁), thereby restoring the bivalent presentation observed in the native PSGL-1 molecule. Finally, to disable Fc receptor binding and complement fixation effect or functions, two amino acids of the IgG-Fc region are mutated (see Example 1).

To increase the avidity of a P-selectin antagonist, a tetrameric form of PSGL-1 can be constructed by truncating the NH₂ 47 amino acids of native PSGL-1 fusing them to both the light and heavy chain regions of the Fc portion of human IgG₄ (see Figure 1). Tetrameric PSGL-1 (tetPSGL-1) was shown to have a 5-10 fold greater affinity for P-selectin compared to dimPSGL-1. However, in the experiment described herein, the tetrameric form of PSGL-1, was found to exacerbate cell adhesion, endothelial cell injury, and thrombosis during venous stasis (see Example 2), presumably through the cross-linking of leukocytes, in this experiment. Higher doses of the tetrameric PSGL-1 induced a baseline inflammatory response characterized by an elevation in leukocyte rolling and a reduction in rolling velocity. Therefore, tetrameric forms of PSGL-1 are not preferred for use in the methods of the invention in which inhibition, treatment, or prevention of thrombosis is desired.

The methods of the invention encompass the use of nucleic acid molecules that differ from the nucleotide sequence shown in SEQ ID NO:1 due to degeneracy of the genetic code and thus encode the same PSGL-1 proteins as those encoded by the nucleotide sequence shown in SEQ ID NO:1. In another embodiment, an isolated nucleic acid molecule included in the methods of the invention has a nucleotide sequence encoding a protein having an amino acid sequence shown in SEQ ID NO:2.

The methods of the invention further include the use of allelic variants of human PSGL-1, *e.g*., functional and non-functional allelic variants. Functional allelic variants are naturally occurring amino acid sequence variants of the human PSGL-1 protein that maintain a PSGL-1 activity as described herein, *e.g.*, P-selectin or E-selectin binding. Functional allelic variants will typically contain only conservative substitution of one or more amino acids of SEQ ID NO:2, or substitution, deletion or insertion of non-critical residues in non-critical regions of the protein. Non-functional allelic variants are naturally occurring amino acid sequence variants of the human PSGL-1 protein that do not have a PSGL-1 activity. Non-functional allelic variants will typically contain a non-conservative substitution, deletion, or insertion or premature truncation of the amino acid sequence of SEQ ID NO:2, or a substitution, insertion or deletion in critical residues or critical regions of the protein.

Various aspects of the invention are described in further detail in the following subsections:

### I. Isolated PSGL-1 Proteins, Anti-PSGL-1 Antibodies, and Anti-P-Selectin Antibodies Used in the Methods of the Invention

The methods of the invention include the use of isolated P-selectin ligand proteins, *e*.*g*. PGSL-1 proteins, and biologically active portions thereof, as well as polypeptide fragments suitable for use as immunogens to raise anti-P-selectin ligand antibodies. In one embodiment, native PSGL-1 proteins can be isolated from cells or tissue sources by an appropriate purification scheme using standard protein purification techniques. In another embodiment, PSGL-1 proteins are produced by recombinant DNA techniques. Alternative to recombinant expression, a PSGL-1 protein or polypeptide can be synthesized chemically using standard peptide synthesis techniques.

As used herein, a "biologically active portion" of a-PSGL-1 protein includes a fragment of a PSGL-1 protein having a PSGL-1 activity. Biologically active portions of a PSGL-1 protein include peptides comprising amino acid sequences sufficiently identical to or derived from the amino acid sequence of the PSGL-1 protein, *e.g.*, the amino acid sequence shown in SEQ ID NO:2, which include fewer amino acids than the full length PSGL-1 proteins, and exhibit at least one activity of a PSGL-1 protein. Typically, biologically active portions comprise a domain or motif with at least one activity of the PSGL-1 protein (*e.g.*, a fragment containing the extracellular domain of PSGL-1, or a fragment thereof, which is capable of interacting with P-selectin and/or E-selectin). A biologically active portion of a PSGL-1 protein can be a polypeptide which is, for example, 18, 20, 22, 25, 50, 75, 100, 125, 150, 175, 200, 250, 300 or more amino acids in length. Biologically active portions of a PSGL-1 protein can be used as targets for developing, agents which modulate a PSGL-1 activity.

In a preferred embodiment, the PSGL-1 protein used in the methods of the invention has at least an extracellular domain of the amino acid sequence shown in SEQ ID NO:2 or P-selectin binding fragment of the extracellular domain of PSGL-1, or an extracellular domain of SEQ ID NO:2. In other embodiments, the PSGL-1 protein is substantially identical to SEQ ID NO:2, and retains the functional activity of the protein of SEQ ID NO:2, yet differs in amino acid sequence due to natural allelic variation or mutagenesis, as described in detail in subsection II below. Accordingly, in another embodiment, the PSGL-1 protein used in the methods of the invention is a protein which comprises an amino acid sequence at least about 50%; 55%, 60%, 65%; 70%, 75%, 80%, 85%, 90%, 91%, 92%, 93%, 94%, 95%, 96%, 97%, 98%, 99% or more identical to SEQ ID NO:2.

In a preferred embodiment, the PSGL-1 protein used in the methods of the invention is a soluble P-selectin ligand protein. A DNA encoding a soluble form of the P-selectin ligand protein may be prepared by expression of a modified DNA in which the regions encoding the transmembrane and cytoplasmic domains of the P-selectin ligand protein are deleted and/or a stop codon is introduced 3' to the codon for the amino acid at the carboxy terminus of the extracellular domain. For example, hydrophobicity analysis predicts that the P-selectin ligand protein set forth in SEQ ID NO:2 has a transmembrane domain comprised of amino acids 311 to 332 of SEQ ID NO:2 and a cytoplasmic domain comprised of amino acids 333 to 402 of SEQ ID NO:2. A modified DNA as described above may be made by standard molecular biology techniques, including site-directed mutagenesis methods which are known in the art or by the polymerase chain reaction using appropriate oligonucleotide primers. Methods for producing several DNAs encoding various soluble P-selectin ligand proteins are set forth in U.S. Patent No. 5,827,817, incorporated herein by reference.

To determine the percent identity of two amino acid sequences or of two nucleic acid sequences, the sequences are aligned for optimal comparison purposes (*e.g.*, gaps can be introduced in one or both of a first and a second amino acid or nucleic acid sequence for optimal alignment and non-identical sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, or 90% of the length of the reference sequence (*e.g.*, when aligning a second sequence to the PSGL-1 amino acid sequence of SEQ ID NO:2 having 400 amino acid residues, at least 280, preferably at least 240, more preferably at least 200, even more preferably at least 160, and even more preferably at least 120, 80, or 40 or more amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch (*J*. *Mol. Biol.* 48:444-453 (1970)) algorithm which has been incorporated into the GAP program in the GCG software package (available at http://www.gcg.com), using either a Blosum 62 matrix or a PAM250 matrix, and a gap weight of 16, 14, 12, 10, 8, 6, or 4 and a length weight of 1, 2, 3,4, 5, or 6. In yet another preferred embodiment, the percent identity between two nucleotide sequences is determined using the GAP program in the GCG software package (available at http/www.gcg.com), using a NWSgapdna.CMP matrix and a gap weight of 40, 50, 60, 70, or 80 and a length weight of 1, 2, 3, 4, 5, or 6. In another embodiment, the percent identity between two amino acid or nucleotide sequences is determined using the algorithm of E. Meyers and W. Miller *(Comput. Appl. Biosci.* 4:11-17 (1988)) which has been incorporated into the ALIGN program (version 2.0 or 2.0U), using a PAM120 weight residue table, a gap length penalty of 12 and a gap penalty of 4.

The methods of the invention may also use PSGL-1 chimeric or fusion proteins. As used herein, a PSGL-1 "chimeric protein" or "fusion protein" comprises a PSGL-1 polypeptide operatively linked to a non-PSGL-1 polypeptide. A "PSGL-1 polypeptide" refers to a polypeptide having an amino acid sequence corresponding to a PSGL-1 molecule, whereas a "non-PSGL-1 polypeptide" refers to a polypeptide having an amino acid sequence corresponding to a protein which is not substantially homologous to the PSGL-1 protein, *e.g.*, a protein which is different from the PSGL-1 protein and which is derived from the same or a different organism. Within a PSGL-1 fusion protein the PSGL-1 polypeptide can correspond to all or a portion of a PSGL-1 protein. In a preferred embodiment, a PSGL-1 fusion protein comprises at least one biologically active portion of a PSGL-1 protein, *e*.*g*., an extracellular domain of PSGL-1 or P-selectin binding fragment thereof. In another preferred embodiment, a PSGL-1 fusion protein comprises at least two biologically active portions of a PSGL-1 protein. Within the fusion protein, the term "operatively linked" is intended to indicate that the PSGL-1 polypeptide and the non-PSGL-1 polypeptide are fused in-frame to each other. The non-PSGL-1 polypeptide can be fused to the N-terminus or C-terminus of the PSGL-1 polypeptide.

For example, in one embodiment, the fusion protein is a recombinant soluble form of PSGL-1 protein in which the extracellular domain of the PSGL-1 molecule is fused to human IgG, *e.g.,* soluble rPSGL-Ig.

In another embodiment, this fusion protein is a PSGL-1 protein containing a heterologous signal sequence at its N-terminus. In certain host cells (*e.g.*, mammalian host cells), expression and/or secretion of PSGL-1 can be increased through use of a heterologous signal sequence.

The soluble PSGL-1 fusion proteins used in the methods of the invention, e.g. rPSGL-Ig, can be incorporated into pharmaceutical compositions and administered to a subject *in vivo.* The soluble PSGL-1 fusion proteins can be used to affect the bioavailability of a PSGL-1 substrate, *e*.*g.*, P-selectin or E-selectin.

Moreover, the PSGL-1-fusion proteins used in the methods of the invention can be used as immunogens to produce anti-P-selectin ligand antibodies in a subject, to purify P-selectin ligands and in screening assays to identify molecules which inhibit the interaction of a P-selectin ligand molecule with a P-selectin molecule.

Preferably, a PSGL-1 chimeric or fusion protein used in the methods of the invention is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, *Current Protocols in Molecular Biology,* eds. Ausubel *et al.* John Wiley & Sons: 1992). Moreover, many expression vectors are commercially available that already encode a fusion moiety (*e*.*g*., a GST polypeptide). A PSGL-1-encoding nucleic acid can be cloned into such an expression vector such that the fusion moiety is linked in-frame to the PSGL-1 protein.

The present invention also pertains to the use of variants of the PSGL-1 proteins which function as either PSGL-1 agonists (mimetics) or as PSGL-1 antagonists. Variants of the PSGL-1 proteins can be generated by mutagenesis, *e.g.*, discrete point mutation or truncation of a PSGL-1 protein. An agonist of the PSGL-1 proteins can retain substantially the same, or a subset, of the biological activities of the naturally occurring form of a PSGL-1 protein. An antagonist of a PSGL-1 protein can inhibit one or more of the activities of the naturally occurring form of the PSGL-1 protein by, for example, competitively modulating a PSGL-1-mediated activity of a PSGL-1 protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the PSGL-1 protein.

In one embodiment, variants of a PSGL-1 protein which function as either PSGL-1 agonists (mimetics) or as PSGL-1 antagonists can be identified by screening combinatorial libraries of mutants, *e.g.*, truncation mutants, of a PSGL-1 protein for PSGL-1 protein agonist or antagonist activity. In one embodiment, a variegated library of PSGL-1 variants is generated by combinatorial mutagenesis at the nucleic acid level and is encoded by a variegated gene library. A variegated library of PSGL-1 variants can be produced by, for example, enzymatically ligating a mixture of synthetic oligonucleotides into gene sequences such that a degenerate set of potential PSGL-1 sequences is expressible as individual polypeptides, or alternatively, as a set of larger fusion proteins (*e.g.*, for phage display) containing the set of PSGL-1 sequences therein. There are a variety of methods which can be used to produce libraries of potential PSGL-1 variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential PSGL-1 sequences. Methods for synthesizing degenerate oligonucleotides are known in the art (see, *e.g.,* Narang, S.A. (1983) *Tetrahedron* 39:3; Itakura *et al*. (1984) *Annu. Rev. Biochem.* 53:323; Itakura *et al. (1984) Science* 198:1056; Ike *et al.* (1983) *Nucleic Acid Res.* 11:477).

In addition, libraries of fragments of a PSGL-1 protein coding sequence can be used to generate a variegated population of PSGL-1 fragments for screening and subsequent selection of variants of a PSGL-1 protein. In one embodiment, a library of coding sequence fragments can be generated by treating a double stranded -PCR fragment of a PSGL-1 coding sequence with a nuclease under conditions wherein nicking occurs only about once per molecule, denaturing the double stranded DNA, renatuting the DNA to form double stranded DNA which can include sense/antisense pairs from different nicked-products, removing single stranded portions from reformed duplexes by treatment with S1 nuclease, and ligating the resulting fragment library into an expression vector. By this method, an expression library can be derived which encodes N-terminal, C-terminal and internal fragments of various sizes of the PSGL-1 protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of PSGL-1 proteins. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique which enhances the frequency of functional mutants in the libraries, can be used in combination with the screening assays to identity PSGL-1 variants (Arkin and Yourvan (1992) *Proc. Natl. Acad. Sci. USA* 89:7811-7815; Delgrave et *al*. (1993) Protein *Engineering* 6(3):327-331).

The methods of the present invention further include the use of anti-PSGL-1 antibodies and anti-P-selectin antibodies. An isolated PSGL-1 protein, or P-selectin protein, or a portion or fragment thereof, can be used as an immunogen to generate antibodies that bind PSGL-1 or P-selectin using standard techniques for polyclonal and monoclonal antibody preparation. P-selectin ligand antibodies are described in, for example, U.S. Patent Number 5,852,175. Antibodies specific for P-selectin are described in, for example, Kurome, T., *et al.* (1994) *J. Biochem.* 115 (3), 608-614.

A full-length PSGL-1 protein or P-selectin protein can be used or, alternatively, antigenic peptide fragments of PSGL-1 or P-selectin can be used as immunogens (Johnston *et al.* (1989) *Cell 56 :* 1033-1044). The antigenic peptide of PSGL-1 comprises at least 8 amino acid residues of the amino acid sequence shown in SEQ ID NO:2 and encompasses an epitope of PSGL-1 such that an antibody raised against the peptide forms a specific immune complex with the PSGL-1 protein. Preferably, the antigenic peptide comprises at least 10 amino acid residues, more preferably at least 15 amino acid residues, even more preferably at least 20 amino acid residues, and most preferably at least 30 amino acid residues.

Preferred epitopes encompassed by the antigenic peptide are regions of PSGL- 1 that are located on the surface of the protein, *e.g.*, hydrophilic regions, as well as regions with high antigenicity.

A PSGL-1 or P-selectin immunogen is typically used to prepare antibodies by immunizing a suitable subject, *(e.g.,* rabbit, goat, mouse, or other mammal) with the immunogen. An appropriate immunogenic preparation can contain, for example, recombinantly expressed PSGL-1 protein or P-selectin protein or a chemically synthesized PSGL-1 or P-selectin polypeptide. The preparation can further include an adjuvant, such as Freund's complete or incomplete adjuvant, or similar immunostimulatory agent Immimization of a suitable subject with an immunogenic PSGL-1 preparation induces a polyclonal anti-PSGL-1 or anti-P-selectin antibody response.

The term "antibody" as used herein refers to immunoglobulin molecules and immunologically active portions of immunoglobulin molecules, *i.e.*, molecules that contain an antigen binding site which specifically binds (immunoreacts with) an antigen, such as a PSGL-1 or P-selectin. Examples of immunologically active portions of immunoglobulin molecules include F(ab) and F(ab')₂ fragments which can be generated by treating the antibody with an enzyme such as pepsin. The invention provides polyclonal and monoclonal antibodies that bind PSGL-1 molecules. The term "monoclonal antibody" or "monoclonal antibody composition", as used herein, refers to a population of antibody molecules that contain only one species of an antigen binding site capable of immunoreacting with a particular epitope of PSGL-1. A monoclonal antibody composition thus typically displays a single binding affinity for a particular PSGL-1 protein or P-selectin with which it immunoreacts. Polyclonal anti-PSGL-1 antibodies or P-selectin antibodies can be prepared as described above by immunizing a suitable subject with a PSGL-1 or P-selectin immunogen. The anti-PSGL-1 antibody or P-selectin antibody titer in the immunized subject can be monitored over time by standard techniques, such as with an enzyme linked immunosorbent assay (ELISA) using immobilized PSGL-1 or P-selectin. If desired, the antibody molecules directed against either antigen can be isolated from the mammal (*e*.*g*., from the blood) and further purified by well known techniques, such as protein A chromatography to obtain the IgG fraction. At an appropriate time after immunization, *e*.*g*., when the antibody titers are highest, antibody-producing cells can be obtained from the subject and used to prepare monoclonal antibodies by standard techniques, such as the hybridoma technique originally described by Kohler and Milstein (1975) *Nature* 256:495-497) (see also, Brown *et al.* (1981) *J. Immunol.* 127:539-46; Brown *et al*. (1980) *J. Biol. Chem.* 255:4980-83; Yeh *et al*. (1976) *Proc*. *Natl. Acad Sci. USA* 76:2927-31; and Yeh *et al.* (1982) *Int. J. Cancer* 29:269-75), the more recent human B cell hyhridoma technique (Kozbor *et al.* (1983) *Immunol Today* 4:72), the EBV-hybridoma technique (Cole *et al.* (1985) *Monoclonal Antibodies* and *Cancer Therapy*, Alan R. Liss, Inc., pp. 77-96) or trioma techniques. The technology for producing monoclonal antibody hybridomas is well known (see generally Kenneth, R. H. in *Monoclonal Antibodies: A New Dimension In Biological Analyses,* Plenum Publishing Corp., New York, New York (1980); Lerner, E. A. (1981) *Yale J. Biol. Med.* 54:387-402; Gefter, M. L. *et al.* (1977) *Somatic Cell Genet.* 3:231-36). Briefly, an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds PSGL-1 or P-selectin.

Any of the many well known protocols used for fosing lymphocytes and immortalized cell lines can be applied for the purpose of generating an anti-PSGL-1 or P-selectin monoclonal antibody (see, *e.g*., *G.* Galfre *et al.* (1977) *Nature* 266:55052; Gefter *et al.* (1977) *supra; Lerner* (1981) *supra;* and Kenneth (1980) supra). Moreover, the ordinarily skilled worker will appreciate that there are many variations of such methods which also would be useful. Typically, the immortal cell line (*e.g*., a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, *e.g.*, the P3-NS1/1-Ag4-1, P3-x63-Ag8.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind PSGL-1 or P-selectin, *e.g.*, using a standard ELISA assay.

Alternative to preparing monoclonal antibody-secreting hybridomas, a monoclonal anti-PSGL-1 or anti-P-selectin antibody can be identified and isolated by screening a recombinant combinatorial immunoglobulin library (e.g., an antibody phage display library) with PSGL-1 or P-selectin respectively to thereby isolate immunoglobulin library members that bind PSGL-1 or P-selectin. Kits for generating and screening phage display libraries are commercially available (*e.g.*, the Pharmacia *Recombinant Phage Antibody System,* Catalog.No. 27-9400-01; and the Stratagene *SurfZAP™ Phage Display Kit,* Catalog No. 240612). Additionally, examples of methods and reagents particularly amenable for use in generating and screening antibody display library can be found in, for example, Ladner *et al.* U.S. Patent No. 5,223,409; Kang ef *al.* PCT International Publication No. WO 92/18619; Dower *et al.* PCT International Publication No. WO 91/17271; *Winter et al.* PCT International Publication WO 92/20791; Markland *et al.* PCT International Publication No. WO 92/15679; Breitling *et al.* PCT International Publication WO 93/01288; McCafferty *et al.* PCT International Publication No. WO 92/01047; Garrard *et al.* PCT International Publication No. WO 92/09690; Ladner *et al.* PCT International Publication No: WO 90/02809; Fuchs *et aL* (1991) *Bio*/*Technology* 9:1370-1372; Hay *et al.* (1992) *Hum. Antibod. Hybridomas* 3:81-85; Huse *et al.* (1989) *Science* 246:1275-1281; Griffiths *et* *al.* (1993) *EMBO J* 12:725-734; Hawkins *et. al.* (1992) *J. Mol. BioL* 226:889-896; Clarkson *et al.* (1991) *Nature* 352:624-628; Gram *et* al. (1992) *Proc. Natl. Acad Sci. USA* 89:3576-3580; Garrad *et al.* (1991) *Bio*/*Technology* 9:1373-1377; Hoogenboom *et al.* (1991) *Nuc. Acid Res.* 19:4133-4137; Barbas *et al.* (1991) *Proc. Natl. Acad. Sci. USA* 88:7978-7982; and McCafferty *et al*. (1990) *Nature* 348:552-554.

Additionally, recombinant anti-PSGL-1 or anti-P-selectin antibodies, such as chimeric and humanized monoclonal antibodies, comprising both human and non-human portions, which can be made using standard recombinant DNA techniques, are within the scope of the methods of the invention. Such chimeric and humanized monoclonal antibodies can be produced by recombinant DNA techniques known in the art, for example using methods described in Robinson *et al.* international Application No. PCT/US86/02269; Akira, *et al.* European Patent Application 184,187; Taniguchi, M., European Patent Application 171;496; Morrison *et al.* European Patent Application 173,494; Neuberger *et al.* PCT International Publication No. WO 86/01533; Cabilly *et al.* U.S. Patent No. 4,816,567; Cabilly *et al*. European Patent Application 125,023; Better *et al.* (1988) *Science* 240:1041-1043; Liu *et al.* (1987) *Proc. Natl. Acad Sci. USA* 84:3439-3443; Liu *et al.* (1987) *J*. *Immunol.* 139:3521-3526; Sun *et al.* (1987) *Proc. Natl. Acad Sci USA* 84:214-218; Nishimura *et al..* (1987) *Canc. Res.-* 47:999-1005; Wood *et, al* (1985) *Nature:* 314:446-443; Shaw *et al.* (1988) *J. Natl. Cancer Inst.* 80:1553-1559; Morrison, S. L. (1985) Science 229:1202-1207; Oi *et al.* (1986) *BioTechniques* 4:214; Winter U.S. Patent 5;225,539; Jones *et al.* (1986) *Nature* 321:552-525; Verhoeyan *et al.* (1988) *Science* 239:1534; and Beidler *et al*. (1988) *J*. *Immunol.* 141:4053-4060.

Antibodies as described herein can be used to detect PSGL-1 protein or P-selectin (*e.g*., in a cellular lysate or cell supernatant) in order to evaluate the abundance and pattern of expression of the protein. Such antibodies can be used diagnostically to monitor protein levels in tissue as part of a clinical testing procedure, e.g., to, for example, determine the efficacy of a given treatment regimen. Detection can be facilitated by coupling (*i.e.,* physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl. chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S, or ³H.

### II. Isolated Nucleic Acid Molecules Used in the Methods of the Invention

The coding sequence of the isolated human PSGL-1 cDNA and the amino acid sequence of the human PSGL-1 polypeptide are shown in SEQ ED NOs:1 and 2, respectively. The PSGL-1 sequence is also described in U.S. Patent Numbers 5,827,817 and 5,843,707, the contents of which are incorporated herein by refrence.

The methods of the invention include the use of isolated nucleic acid molecules that encode PSGL-1 proteins or biologically active portions thereof, as well as nucleic acid fragments sufficient for use as hybridization probes to identify PSGL-1-encoding nucleic acid molecules (e.g., PSGL-1 mRNA) and fragments for use as PCR primers for the amplification or mutation of PSGL-1 nucleic acid molecules. As used herein, the term "nucleic acid molecule" is intended to include DNA molecules (e.g., cDNA or genomic DNA) and RNA molecules (*e.g.*, mRNA) and analogs of the DNA or RNA generated using nucleotide analogs. The nucleic acid molecule can be single-stranded or double-stranded, but preferably is double-stranded DNA.

A nucleic acid molecule used in the methods of the present invention, *e.g.*, a nucleic acid molecule having the nuoleotide sequence of SEQ ID NO:1, or a portion thereof, can be isolated using standard molecular biology techniques and the sequence information provided herein. Using all or portion of the nucleic acid sequence of SEQ ID NO:1 as a hybridization probe, PSGL-1 nucleic acid molecules can be isolated using standard hybridization and cloning techniques (*e.g.*, as described in Sambrook, J., Fritsh, E. F., and Maniatis, T. *Molecular Cloning: A Laboratory Manual. 2nd, ed, Cold Spring Harbor Laboratory,* Cold Spring Harbor Laboratory Press, Cold Spring Harbor, NY, 1989).

Moreover, a nucleic acid molecule encompassing all or a portion of SEQ ID NO:1 can be isolated by the polymerase chain reaction (PCR) using synthetic oligonucleotide primers designed based upon the sequence of SEQ ID NO: 1.

A nucleic acid used in the methods.of the invention can be amplified using cDNA, mRNA or, alternatively, genomic DNA as a template and appropriate oligonucleotide primers according to standard PCR amplification techniques., Furthermore, oligonucleotides corresponding to PSGL-1 nucleotide sequences can be prepared by standard synthetic techniques, *e.g.*, using an automated DNA synthesizer.

In a preferred embodiment, the isolated nucleic acid molecules used in the methods of the invention comprise the nucleotide sequence shown in SEQ ID NO:1, a complement of the nucleotide sequence shown in SEQ ID NO:1; or a portion of any of these nucleotide sequences A nucleic acid molecule which is complementary to the nucleotide sequence shown in SEQ ID NO:1, is one which is sufficiently complementary to the nucleotide sequence shown in SEQ ID NO:1 such that it can hybridize to the nucleotide sequence shown in SEQ ID NO:1 thereby forming a stable duplex.

In still another preferred embodiment, an isolated nucleic acid molecule used in the methods of the present invention comprises a nucleotide sequence which is at least about 55%, 60%, 65%, 70%, 75%, 80%, 85%, 90%, 91%, 92%, 93%, 94%, 95%, 96%, 97%, 98%, 99% or more identical to the entire length of the nucleotide sequence shown in SEQ ID NO:1 or a portion of any of this nucleotide sequence.

Moreover, the nucleic acid molecules used in the methods of the invention can comprise only a portion of the nucleic acid sequence of SEQ ID NO: 1, for example, a fragment which can be used as a probe or primer or a fragment encoding a portion of a PSGL-1 protein, *e.g.*, a biologically active portion of a PSGL-1 protein. The probe/primer typically comprises substantially purified oligonucleotide. The oligonucleotide typically comprises a region of nucleotide sequence that hybridizes under stringent conditions to at least about 12 or 15, preferably about 20 or 25, more preferably about 30, 35, 40, 45, 50, 55, 60, 65, or 75 consecutive nucleotides of a sense sequence of SEQ ID NO: 1 of an anti-sense sequence of SEQ ID NO:1 or of a naturally occurring allelic variant or mutant of SEQ ID NO: 1. In one embodiment, a nucleic acid molecule used in the methods of the present invention comprises a nucleotide sequence which is greater than 100, 100-200, 200-300, 300-400, 400-500, 500-600, 600-700, 700-800, 800-900, 900-1000, 1000-1100, 1100-1200, 1200-1300, 1300-1400, 1400-1500, 1500-1600, or more nucleotides in length and hybridizes under stringent hybridization conditions to a nucleic acid molecule of SEQ ID NO:1.

As used herein, the term "hybridizes under stringent conditions" is intended to describe conditions for hybridization and washing under which nucleotide sequences that are significantly identical or homologous to each other remain hybridized to each other. Preferably, the conditions are such that sequences at least about 70%, more preferably at least about 80%, even more preferably at least about 85% or 90% identical to each other remain hybridized to each other. Such stringent conditions are known to those skilled in the art and can be found in *Current Protocols in Molecular Biology,* Ausubel *et al.,* eds., John Wiley & Sons, Inc. (1995), sections 2, 4 and 6. Additional stringent conditions can be found in *Molecular Cloning: A Laboratory Manual,* Sambrook *et al.,* Cold Spring Harbor Press, Cold Spring Harbor, NY (1989), chapters 7, 9 and 11. A preferred, non-limiting example of stringent hybridization conditions includes hybridization in 4X sodium chloride/sodium citrate (SSC), at about 65-70°C (or hybridization in 4X SSC plus 50% formamide at about 42-50°C) followed by one or more washes in 1X SSC, at about 65-70°C. A preferred, non-limiting example of highly stringent hybridization conditions includes hybridization in 1X SSC, at about 65-70°C (or hybridization in 1X SSC plus 50% formamide at about 42-50°C) followed by one or more washes in 0.3X SSC, at about 65-70°C. A preferred, non-limiting example of reduced stringency hybridization conditions includes hybridization in 4X SSC, at about 50-60°C (or alternatively hybridization in 6X SSC plus 50% formamide at about 40-45° C) followed by one or more washes in 2X SSC, at about 50-60°C. Ranges intermediate to the above-recited values, *e*.*g*., at 65-70°C or at 42-50°C are also intended to be encompassed by the present invention. SSPE (1xSSPE is 0.15M NaCl, 10mM NaH₂PO₄, and 1.25mM EDTA, pH 7.4) can be substituted for SSC (1xSSC is 0.15M NaCl and 15mM sodium citrate) in the hybridization and wash buffers; washes are performed for 15 minutes each after hybridization is complete. The hybridization temperature for hybrids anticipated to be less than 50 base pairs in length should be 5-10°C less than the melting temperature (Tₘ) of the hybrid, where Tₘ is determined according to the following equations. For hybrids less than 18 base pairs in length, Tₘ(°C) = 2(# of A + T bases) + 4(# of G + C bases). For hybrids between 18 and 49 base pairs in length, Tₘ(°C) = 81.5 + 16.6(log_{10[}Na⁺]) + 0.41(%G+C) - (600/N), where N is the number of bases in the hybrid; and [Na⁺] is the concentration of sodium ions in the hybridization buffer ([Na⁺] for 1xSSC = 0.165 M). It will also be recognized by the skilled practitioner that additional reagents may be added to hybridization and/or wash buffers to decrease non-specific hybridization of nucleic acid molecules to membranes, for example; nitrocellulose or nylon membranes, including but not limited to blocking agents (*e.g.*, BSA or salmon or herring sperm carrier DNA), detergents (*e.g.*, SDS), chelating agents (*e.g.*, EDTA), Ficoll, PVP and the like. When using nylon membranes, in particular, an additional preferred, non-limiting example of stringent hybridization conditions is hybridization in 0.25-0.5M NaH₂PO₄, 7%-SDS at about 65°C, followed by one or more washes at 0.02M NaH₂PO₄, 1% SDS at 65°C, see *e*.*g*., Church and Gilbert (1984) *Proc, Natl. Acad Sci. USA* 81:1991-1995, (or alternatively 0.2X SSC, 1% SDS).

Preferably, an isolated nucleic acid molecule of the invention that hybridizes under stringent conditions to the sequence of SEQ ID NO:1 corresponds to a naturally-occurring nucleic acid molecule. As used herein, a "naturally-occurring" nucleic acid molecule refers to an RNA or DNA molecule having a nucleotide sequence that occurs in nature (*e.g.*, encodes a natural protein).

In preferred embodiments, the probe further comprises a label group attached thereto, *e.g.*, the label group can be a radioisotope, a fluorescent compound, an enzyme, or an enzyme co-factor. Such probes can be used as a part of a diagnostic test kit for identifying cells or tissue which misexpress a PSGL-1 protein, such as by measuring a level of a PSGL-1-encoding nucleic acid in a sample of cells from a subject *e*.*g*., detecting. PSGL-1 mRNA levels or determining whether a genomic PSGL-1 gene has been mutated or deleted.

The methods of the present invention may use non-human orthologues of the human PSGL-1 protein. Orthologues of the human PSGL-1 protein are proteins that are isolated from non-human organisms and possess the same PSGL-1 activity.

The methods of the present invention further include the use of nucleic acid molecules comprising the nucleotide sequence of SEQ ID NO:1 or a portion thereof, in which a mutation has been introduced. The mutation may lead to amino acid substitutions at "non-essential" amino acid residues or at "essential" amino acid residues. A "non-essential" amino acid residue is a residue that can be altered from the wild-type sequence of PSGL-1 (*e.g.*, the sequence of SEQ ID NO:2) without altering the biological activity, whereas an "essential" amino acid residue is required for biological activity. For example, amino acid residues comprising fragments which are capable of interacting with P-selectin or which are capable of inhibiting P-selectin-mediated cellular adhesion or cellular migration are not likely to be amenable to alteration.

Mutations can be introduced into SEQ ID NO:1 by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (*e.g*., lysine, arginine, histidine), acidic side chains (*e.g.*, aspartic acid, glutamic acid), uncharged polar side chains (*e.g.*, asparagine, glutamine, serine, threonine, tyrosine, cysteine), nonpolar side chains (*e.g.*, glycine, alanine, valine, leucine, isoleucine, proline, phenylalanine, methionine, tryptophan), beta-branched side chains (*e.g.*; threonine, valine, isoleucine) and aromatic side chains (*e.g.*, tyrosine, phenylalanine, tryptophan, histidine). Thus, a predicted nonessential amino acid residue in a PSGL-1 protein is preferably replaced with another amino acid residue from the same side chain family. Alternatively, in another embodiment, mutations can be introduced randomly along all or part of a PSGL-1 coding sequence, such as by saturation mutagenesis, and the resultant mutants can be screened for PSGL-1 biological activity to identify mutants that retain activity. Following mutagenesis of SEQ ID NO:1 the encoded protein can be expressed recombinantly and the activity of the protein can be determined using the assay described herein.

Given the coding strand sequences encoding PSGL-1 disclosed herein, antisense nucleic acids of the invention can be designed according to the rules of Watson and Crick base pairing. The antisense nucleic acid molecule can be complementary to the entire coding region of PSGL-1 mRNA, but more preferably is an oligonucleotide which is antisense to only a portion of the coding or noncoding region of PSGL-1 mRNA. For example, the antisense oligonucleotide can be complementary to the region surrounding the translation start site of PSGL-1 mRNA. An antisense oligonucleotide can be, for example, about 5, 10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid (*e.g.*, an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e.g*., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid(v), 5-methyl-2-thiouracil, 3-(3-amino-3-N-2-carboxypropyl) uracil, (acp3)w, and 2,6-diaminopurine. Alternatively, the antisense nucleic add can be produced biologically using an expression vector into which a nucleic acid has been subcloned in an antisense orientation *(i.e.,* RNA transcribed from the inserted nucleic acid will be of an antisense orientation to a target nucleic acid of interest, described further in the following subsection).

In yet another embodiment, the PSGL-1 nucleic acid molecules used in the methods of the present invention can be modified at the base moiety, sugar moiety or phosphate backbone to improve, *e.g.,* the stability, hybridization, or solubility of the molecule. For example, the deoxyribose phosphate backbone of the nucleic acid molecules can be modified to generate peptide nucleic acids (see Hyrup B. *et al.* (1996) *Bioorganic & Medicinal Chemistry* 4(1): 5-23). As used herein, the terms "peptide nucleic acids" or "PNAs" refer to nucleic acid mimics, *e.g.*, DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup B. *et al.* (1996) *supra;* Perry-O'Keefe *et al.* (1996) *Proc*. *Natl. Acad. Sci.* 93:14670-675.

PNAs of PSGL-1 nucleic acid molecules can be used in the therapeutic and diagnostic applications described herein. For example, PNAs can be used as antisense or antigene agents for sequence-specific modulation of gene expression by, for example, inducing transcription or translation arrest or inhibiting replication. PNAs of PSGL-1 nucleic acid molecules can also be used in the analysis of single base pair mutations in a gene, (*e.g.*, by PNA-directed PCR clamping); as artificial restriction enzymes' when used in combination with other enzymes, *(e.g.,* S1 nucleases (Hyrup B. *et al.* (1996) *supra));* or as probes or primers for DNA sequencing or hybridization (Hyrup B. *et al.* (1996) supra; Perry-O'Keefe *et al.* (1996) *supra).*

In another embodiment, PNAs of PSGL-1 can be modified, (*e.g.*, to enhance their stability or cellular uptake), by attaching lipophilic or other helper groups to PNA, by the formation of PNA-DNA chimeras, or by the use of liposomes or other techniques of drug delivery known in the art. For example, PNA-DNA chimeras of PSGL-1 nucleic acid molecules can be generated which may combine the advantageous properties of PNA and DNA. Such chimeras allow DNA recognition enzymes, (*e.g.*, RNAse H and DNA polymerases), to interact with the DNA portion while the PNA portion would provide high binding affinity and specificity. PNA-DNA chimeras can be linked using.linkers of appropriate lengths selected in terms of base stacking, number of bonds between the nucleobases, and orientation (Hyrup B. *et al.* (1996) *supra).* The synthesis of PNA-DNA chimeras can be performed as described in Hyrup B. *et al.* (1996) *supra* and Finn P.J. *et al.* (1996) *Nucleic Acids Res.* 24 (17): 3357-63. For example, a DNA chain can be synthesized on a solid support using standard phosphoramidite coupling chemistry and modified nucleoside analogs, *e.g.*, 5'-(4-methoxytrityl)amino-5'-deoxy-thymidine phosphoramidite, can be used as a between the PNA and the 5' end of DNA (Mag, M. *et al.* (1989) *Nucleic Acid Res.* 17:5973-88). PNA monomers are then coupled in a stepwise manner to produce a chimeric molecule with a 5' PNA segment and a 3' DNA segment (Finn P.J. *et al.* (1996) *supra).* Alternatively, chimeric molecules can be synthesized with a 5' DNA segment and a 3' PNA segment (Peterser, K.H. *et al.* (1975) *Bioorganic Med Chem. Lett.* 5: 1119-11124).

In other embodiments, the oligonucleotide used in the methods of the invention may include other appended groups such as peptides (*e.g*., for targeting host cell receptors *in vivo),* or agents facilitating transport across the cell membrane (see, *e.g.,* Letsinger *et al.* (1989) *Proc. Natl. Acad. Sci. USA* 86:6553-6556; Lemaitre *et al.* (1987) *Proc. Natl. Acad Sci. USA* 84:648-652; PCT Publication No. W088/09810) or the blood-brain barrier (*see*, *e*.*g*., PCT Publication No. W089/10134). In addition, oligonucleotides can be modified with hybridization-triggered cleavage agents (See, *e.g*., Krol *et al.* (1988) *Bio-Techniques* 6:958-976) or intercalating agents. (See, *e*.*g*., Zon (1988) *Pharm. Res.* 5:539-549): To this end, the oligonucleotide may be conjugated to another molecule, (*e.g.*, a peptide, hybridization triggered cross-linking agent, transport agent, or hybridization-triggered cleavage agent).

A DNA encoding other fragments and altered forms of P-selectin ligand protein used in the methods of the invention may be prepared by expression of modified DNAs in which portions of the full-length sequence have been deleted or altered. Substantial deletions of the P-selectin ligand protein sequence can be made while retaining P-selectin ligand protein activity. For example, P-selectin ligand proteins comprising the sequence from amino acid 42 to amino acid 189 of SEQ ID NO: 2, the sequence from amino acid 42 to amino acid 118 of SEQ ID NO:2, or the sequence from amino acid 42 to amino acid 89 of SEQ ID NO: 2 each retain the P-selectin protein binding activity and the ability to bind to E-selectin. P-selectin ligand proteins in which one or more N-linked glycosylation sites (such as those at amino acids 65, 111 and 292 of SEQ ID NO: 2) have been changed to other amino acids or deleted also retain P-selectin protein binding activity and the ability to bind E-selectin. P-selectin ligand proteins comprising from amino acid 42 to amino acid 60 of SEQ ID NO:2 (which includes a highly anionic region of the protein from amino acid 45 to amino acid 58 of SEQ ID NO:2) also retain P-selectin ligand protein activity; however, P-selectin ligand proteins limited to such sequence do not bind to E-selectin. Preferably, a P-selectin ligand protein retains at least one (more preferably at least two and most preferably all three) of the tyrosine residues found at amino acids 46, 48 and 51 of SEQ ID NO: 2, sulfation of which may contribute to P-selectin ligand protein activity. Construction of DNAs encoding these and other active fragments or altered forms of P-selectin ligand protein may be accomplished in accordance with methods known to those skilled in the art.

The isolated DNA used in the methods of the invention may be operably linked to an expression control sequence such as the pMT2 or pED expression vectors disclosed in Kaufman *et al.,* Nucleic Acids Res.:19, 4485-4490 (1991), in order to produce the P-selectin ligand recombinantly. Many suitable expression control sequences are known in the art. General methods of expressing recombinant proteins are also known and are exemplified in R. Kaufman, Methods in Enzymology 185, 537-566 (1990). As defined herein "operably linked" means enzymatically or chemically ligated to form a covalent bond between the isolated DNA of the invention and the expression control sequence, in such a way that the P-selectin ligand protein is expressed by a host cell which has been transformed (transfected) with the ligated DNA/expression control sequence.

Several endoproteolytic enzymes are known which cleave precursor peptides at the carboxyl side of paired amino acid sequences (e.g., -Lys-Arg- and -Arg-Arg-) to yield mature proteins. Such enzymes are generally known as paired basic amino acid converting enzymes or PACE, and their use in recombinant production of mature peptides is extensively disclosed in WO 92/09698 and U.S. Application Serial No. 07/885,972, both of which are incorporated herein by reference. The PACE family of enzymes are known to increase the efficiency of proteolytic processing of precursor polypeptides in recombinant host cells. As mentioned above, the P-selectin ligand protein of the invention contains such a PACE cleavage site.

The soluble mature P-selectin ligand protein used in the methods of the invention may be made by a host cell which contains a DNA sequence encoding any soluble P-selectin ligand protein as described herein and a DNA sequence encoding PACE as described in WO 92/09698 and U.S. Application Serial No. 07/885,972, incorporated herein by reference. Such a host cell may contain the DNAs as the result of co-transformation or sequential transformation of separate expression vectors containing the soluble P-selectin ligand protein DNA and the PACE DNA, respectively. A third DNA which encodes a 3/4FT may also be co-transfotmed with the DNAs encoding the P-selectin ligand protein and PACE. Alternatively, the host cell may contain the DNAs as the result of transformation of a single expression vector containing both soluble P-selectin ligand protein DNA and PACE DNA. Construction of such expression vectors is within the level of ordinary skill in molecular biology. Methods for co-transformation and transformation are also known.

Many DNA sequences encoding PACE are known. For example, a DNA encoding one form of PACE, known as furin, is disclosed in A.M.W. van den Ouweland *et al*., Nucl. Acids Res. 18, 664 (1990), incorporated herein by reference. A cDNA encoding a soluble form of PACE, known as PACESOL, is set forth in SEQ ID NO:5. DNAs encoding other forms of PACE also exist, and any such PACE-encoding DNA may be used to produce the soluble mature P-selectin ligand protein of the invention, so long as the PACE is capable of cleaving the P-selectin ligand protein at amino acids 38-41. Preferably, a DNA encoding a soluble form of PACE is used to produce the soluble mature P-selectin ligand protein of the present invention.

The DNAs encoding a soluble form of the P-selectin ligand protein and PACE, separately or together; may be operably linked to an expression control sequence such as those contained in the pMT2 or pED expression vectors discussed above, in order to produce the PACE-cleaved soluble P-selectin ligand recombinantly. Additional suitable expression control sequences are known in the art.

### III Recombinant Expression Vectors and Host Cells Used in the Methdds of the Invention

The methods of the invention *(e.g.,* the screening assays. described herein) include the use of vectors, preferably expression vectors, containing a nucleic acid encoding. a PSGL-1 protein (or a portion thereof). As used herein, the term "vector" refers to a nucleic acid molecule capable of transporting another nucleic acid to which it has been linked. One type of vector is a "plasmid", which refers to a circular double stranded DNA loop, into which additional DNA segments can be ligated. Another type of vector is a viral vector, wherein additional DNA segments can- be ligated into the viral genome. Certain vectors are capable of autonomous replication in a host cell into which they are introduced (*e.g.*, bacterial vectors having a bacterial origin of replication and episomal mammalian vectors). Other vectors (*e.g.*, non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e.g.*, replication defective retroviruses, adenoviruses and adeno-associated viruses), which serve equivalent functions.

The recombinant expression vectors to be used in the methods of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence *(e.g.,* in an *in vitro* transcription/translation system or in a host cell when the vector is introduced into the host cell). The term "regulatory sequence" is intended to include promoters, enhancers and other expression control elements (*e.g.*, polyadenylation signals). Such, regulatory sequences are described, for example, in Goeddel (1990) *Methods Enzymol.* 185:3-7. Regulatory sequences include those which direct constitutive expression of a nucleotide sequence in many types of host cells and those which direct expression of the nucleotide sequence only in certain host cells (*e.g.*, tissue-specific regulatory sequences). It will be appreciated by those skilled in the art that the design of the expression vector can depend on such factors as the choice of the host cell to be transformed, the level of expression of protein desired, and the like. The expression vectors of the invention can be introduced into host cells to thereby produce proteins or peptides, including fusion proteins or peptides, encoded by nucleic acids as described herein (*e.g.*, PSGL-1 proteins, mutant forms of PSGL-1 proteins, fusion proteins, and the like). The recombinant expression vectors to be used in the methods of the invention can be designed for expression of P-selectin ligand proteins in prokaryotic or eukaryotic cells. For example, PSGL-1 proteins can be expressed in bacterial cells such as *E. coli,* insect cells (using baculovirus expression vectors), yeast cells, or mammalian cells. Suitable host cells are discussed further in Goeddel (1990) *supra*. Alternatively, the recombinant expression vector can be transcribed and translated *in vitro,* for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K.S. (1988) *Gene* 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be utilized in PSGL-1 activity assays, (*e.g.,* direct assays or competitive assays described in detail below), or to generate antibodies specific for PSGL-1 proteins.

In another embodiment, a nucleic acid of the invention is expressed in mammalian cells using a mammalian expression vector. Examples of mammalian expression vectors include pCDM8. (Seed, B. (1987) *Nature* 329:840) and pMT2PC (Kaufman *et al.* (1987) *EMBO J*.6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, J. *et al., Molecular Cloning: A Laboratory Manual. 2nd ed.*, *Cold Spring Harbor Laboratory,* Cold Spring Harbor Laboratory Press, Cold Spring Harbor, NY, 1989.

In another embodiment, the recombinant mammalian expression vector is capable of directing expression of the nucleic acid preferentially in a particular cell type (*e.g.*, tissue-specific regulatory elements are used to express the nucleic acid).

The methods of the invention may further use a recombinant expression vector comprising a DNA molecule of the invention cloned into the expression vector in an antisense orientation. That is the DNA molecule is operatively linked to a regulatory sequence in a manner which allows for expression (by transcription of the DNA molecule) of an RNA molecule which is antisense to PSGL-1 mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific, or cell type specific expression of antisense RNA. The antisense expression vector can be in the form of a recombinant plasmid, phagemid, or attenuated virus in which antisense nucleic acids are produced under the control of a high efficiency regulatory region, the activity of which can be determined by the cell type into which the vector is introduced. For a discussion of the regulation of gene expression using antisense genes, see Weintraub, H. *et al*., Antisense RNA as a molecular tool for genetic analysis, *Reviews - Trends in Genetics,* Vol. 1(1) 1986.

Another aspect of the invention pertains to use of host cells into which a PSGL-1 nucleic acid molecule of the invention is introduced, *e.g*., a PSGL-1 nucleic acid molecule within a recombinant expression vector or a PSGL-1 nucleic acid molecule containing sequences which allow it to homologously recbmbine into a specific site of the host cell's genome. The terms "host cell" and "recombinant host cell" are used interchangeably herein. It is understood that such terms refer not only to the particular subject cell but to the progeny or potential progeny of such a cell Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a PSGL-1 protein can be expressed in bacterial cells such as *E*. *coli,* insect cells, yeast or mammalian cells (such as Chinese hamster ovary cells (CHO) or COS cells). Other suitable host cells are known to those skilled in the art.

A number of types of cells may act as suitable host cells for expression of the P-selectin ligand protein. Suitable host cells are capable of attaching carbohydrate side chains characteristic of functional P-selectin ligand protein. Such capability may arise by virtue of the presence of a suitable glycosylating enzyme within the host cell, whether naturally occurring, induced by chemical mutagenesis, or through transfection of the host cell with a suitable expression plasmid containing a DNA sequence encoding the glycosylating enzyme. Host cells include, for example, monkey COS cells, Chinese Hamster Ovary (CHO) cells, human kidney 293 cells, human epidermal A431 cells, human Colo205 cells, 3T3 cells, CV-1 cells, other transformed primate cell lines, normal diploid cells, cell strains derived from *in vitro* culture of primary tissue, primary explants, HeLa cells, mouse L cells, BHK, HL-60, U937, or HaK cell.

The P-selectin ligand protein may also be produced by operably linking the isolated DNA of the invention and one or more DNAs encoding suitable glycosylating enzymes to suitable control sequences in one or more insect expression vectors, and employing an insect expression system. Materials and methods for baculovirus/insect cell expression systems are commercially available in kit form from, *e.g.*, Invitrogen, San Diego, California, U.S.A. (the MaxBac® kit), and such methods are well known in the art, as described in Summers and Smith, Texas Agricultural Experiment Station Bulletin No. 1555 (1987), incorporated herein by reference. Solution forms of the P-selectin ligand protein may also be produced in insect cells using appropriate isolated DNAs as described above. A DNA encoding a form of PACE may further be co-expressed in an insect host cell to produce a PACE-cleaved form of the P-selectin ligand protein.

Alternatively, it may be possible to produce the P-selectin ligand protein in lower eukaryotes such as yeast or in prokaryotes such as bacteria. Potentially suitable yeast strains include *Saccharomyces cerevisiae, Schizosaccharomyces pombe, Kluyveromyces* strains, *Candida,* or any yeast strain capable of expressing heterologous proteins. Potentially suitable bacterial strains include *Escherichia coli, Bacillus subtilis, Salmonella typhimurium,* or any bacterial strain capable of expressing heterologous proteins. If the P-selectin ligand protein is made in yeast or bacteria, it is necessary to attach the appropriate carbohydrates to the appropriate sites on the protein moiety covalently, in order to obtain the glycosylated P-selectin ligand protein. Such covalent attachments may be accomplished using known chemical or enzymatic methods.

Vector DNA can be introduced into prokaryotic or eukaryotic cells via conventional transformation or transaction techniques. As used herein, the terms "transformation" and "transfection" are intended to refer to a variety of art-recognized techniques for introducing foreign nucleic acid (*e.g*., DNA) into a host cell, including calcium phosphate or calcium chloride co-precipitation, DEAE-dextran-mediated transfection, lipofection, or electroporation. Suitable methods for transforming or transfecting host cells can be found in Sambrook *et al.* (*Molecular Cloning: A Laboratory Manual 2nd, ed., Cold Spring Harbor Laboratory,* Cold Spring Harbor Laboratory Press, Cold Spring Harbor, NY, 1989), and other laboratory manuals.

A host cell used in the methods of the invention, such as a prokaryotic or eukaryotic host cell in culture, can be used to produce (*i.e.*, express) a PSGL-1 protein. Accordingly, the invention further provides methods for producing a PSGL-1 protein using the host cells of the invention. In one embodiment, the method comprises culturing the host cell of the invention (into which it recombinant expression vector encoding a PSGL-1 protein has been introduced) in a suitable medium such that a PSGL-1 protein is produced. In another embodiment, the method further comprises isolating a PSGL-1 protein from the medium or the host cell.

### IV. Methods of Treatment or Prevention of Thrombosis:

The present invention provides for both prophylactic and therapeutic methods of treating a subject, *e.g.*, a human, at risk of (or susceptible to) thrombosis, including DVT. With regard to both prophylactic and therapeutic methods of treatment, such treatments may be specifically tailored or modified, based on knowledge obtained from the field of pharmacogenomics. "Pharmacogenomics," as used herein, refers to the application of genomics technologies such as gene sequencing, statistical genetics, and gene expression analysis to drugs in clinical development and on the market. More specifically, the term refers to the study of how a patient's genes determine his or her response to a drug (*e.g.*, a patient's "drug response phenotype", or "drug response genotype").

Thus, another aspect of the invention provides methods for tailoring a subject's prophylactic or therapeutic treatment with either the P-selectin antagonists of the present invention or P-selectin ligand modulators according to that individual's drug response genotype. Pharmacogenomics allows a clinician or physician to target prophylactic or therapeutic treatments to patients who will most benefit from and to avoid treatment of patients who will experience toxic drug-related side effects.

### A. Prophylactic And Therapeutic Methods

In one aspect, the invention provides a method for modulating, *e.g.*, inhibiting, treating, or preventing thrombosis in a subject by administering to the subject a composition which includes an agent which modulates PSGL-1 expression or PSGL-1 activity, *e.g.*, modulate P-selectin or E-selectin binding, modulates cellular adhesion, *e.g.*, cell to cell adhesion (*e.g.*, leukocyte-endothelial cell or leukocyte-platelet adhesion) and cell (*e.g.*, platelet or leukocyte) adhesion to blood vessels, modulates cell (*e.g.*, leukocyte or platelet) migration, *e.g.*, movement relative to blood vessels, modulates leukocyte rolling velocity, and modulates thrombosis. Subjects at risk for thrombosis can be identified by, for example, any or a combination of the diagnostic or prognostic assays described herein or known by one of skill in the art. In particular, subjects at risk for thrombosis are those individuals who suffer from cardiovascular disease. Subjects who are at risk for thrombosis also include those who are undergoing cardiovascular and general vascular procedures or intervention such as surgical revascularization, stenting, PCTA or other intervention, surgical or non-surgical, which causes vascular injury. Subjects at risk for thrombosis, including deep vein thrombosis, include those who have undergone any type of surgical procedure. Moreover, subjects at risk for thrombosis include subjects who are subjected to prolonged immobilization.

Cardiovascular diseases and disorders which place a subject at risk for thrombosis and make them a target for treatment with the P-selectin antagonists of the invention include arteriosclerosis, ischemia reperfusion injury, arterial inflammation, rapid ventricular pacing, aortic bending, vascular heart disease, atrial fibrillation, congestive heart failure, sinus node dysfunction, angina, heart failure, hypertension, atrial fibrillation, atrial-flutter, or cardiomyopathy, *e*.*g*., dilated cardiomyopathy and idiopathic cardiomyopathy, myocardial infarction, coronary artery disease, coronary artery spasm, and arrhythmia.

Administration of a prophylactic or theraputic agent, *e.g*., a P-selectin ligand molecule, or a fragment thereof having P-selectin ligand activity, *e*.*g*., soluble PSGL-1, or a soluble recombinant PSGL fusion protein, *e.g*., dimeric PSGL-1 (also referred to herein as rPSGL-Ig), anti-P-selectin antibodies or biologically active fragments thereof, or anti-P-selectin ligand antibodies or biologically active fragments thereof, can occur prior to the manifestation of thrombosis, such that thrombosis is inhibited or, alternatively, delayed in its progression.

Methods of administering to a subject a P-selectin antagonist, *e.g*., an anti-P-selectin antibody, an anti-P-selectin ligand antibody, soluble P-selectin ligand, soluble PSGL-1, or fragments thereof, or soluble rPSGL-Ig, to prevent or treat thrombosis, include, but are not limited to, the following methods.

The soluble P-selectin antagonist of the invention are administered to a subject in the form of a pharmaceutical composition suitable for such administration. Such compositions typically include an effective amount of the active agent (*e.g.*, protein or antibody) and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition used in the therapeutic methods of the invention is formulated to be compatible with its intended route of administration. Examples of routes of administration include parenteral, *e.g.*, intravenous, intradermal, subcutaneous, oral (*e.g.*, inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the agent that modulates PSGL-1 activity (*e.g.*, a fragment of a soluble PSGL-1 protein) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired, ingredient from a previously sterile-altered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form, of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e*.*g.*, a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The agents that modulate PSGL-1 activity can also be prepared in the form of suppositories (*e.g.*, with conventional suppository bases such as cocoa butter and other glycerides) or retention enemas for rectal delivery.

In one embodiment, the agents that modulate PSGL-1 activity are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, form example, described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the agent that modulates PSGL-1 activity and the particular therapeutic effect to be achieved, and the limitations inherent in the art of compounding such an agent for the treatment of subjects.

Toxicity and therapeutic efficacy of such agents can be determined by standard pharmaceutical procedures in cell cultures or experimental animals, *e.g.,* for determining the LD50 (the dose lethal to 50% of the population) and the ED50 (the dose therapeutically effective in 50% of the population). The dose ratio between toxic and therapeutic effects is the therapeutic index and can be expressed as the ratio LD50/ED50. Agents which exhibit large therapeutic indices are preferred. While agents that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage for use in humans. The dosage of such PSGL-1 modulating agents lies preferably within a range of circulating concentrations that include the ED50 with little or no toxicity. The dosage may vary within this range depending upon the dosage form employed and the route of administration utilized. For any agent used in the therapeutic methods of the invention, the therapeutically effective dose can be estimated initially from cell culture assays. A dose may be formulated in animal models to achieve a circulating plasma concentration range that includes the IC50 (*i*.*e*., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of protein or polypeptide (*i*.*e*., an effective dosage) ranges from about 0.001 to 30 mg/kg body weight, preferably about 0.01.to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

In a preferred example, a subject is treated with antibody, protein, or polypeptide in the range of between about 0.1 to 20 mg/kg body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage of antibody, protein, or polypeptide used for treatment may increase or decrease over the course of a particular treatment. Changes in dosage may result and become apparent from the results of diagnostic assays as described herein.

The present invention encompasses agents which modulate expression or activity. An agent may, for example, be a small molecule. For example, such small molecules include, but are not limited to, peptides, peptidomimetics, amino acids, amino acid analogs, polynucleotides, polynucleotide analogs, nucleotides, nucleotide analogs, organic or inorganic compounds *(i.e., including* heteroorganic and organometallic compounds) haying a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds. It is understood that appropriate doses of small molecule agents depends upon a number of factors within the ken of the ordinarily skilled physician, veterinarian, or researcher. The dose(s) of the small molecule will vary, for example, depending upon the identity, size, and condition of the subject or sample being treated, further depending upon the route by which the composition is to be administered, if applicable, and the effect which the practitioner desires the small molecule to have upon the nucleic acid or polypeptide of the invention.

Exemplary doses include milligram or microgram amounts of the small molecule per kilogram of subject or sample weight (*e.g.*, about 1 microgram per kilogram to about 500 milligrams per kilogram, about 100 micrograms per kilogram to about 5 milligrams per kilogram, or about 1 microgram per kilogram to about 50 micrograms per kilogram). It is furthermore understood that appropriate doses of a small molecule depend upon the potency of the small molecule with respect to the expression or activity to be modulated. Such appropriate doses may be determined using the assays described herein. When one or more of these small molecules is to be administered to an animal (*e.g.*, a human) in order to modulate expression or activity of a polypeptide or nucleic acid of the invention, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

Further, an antibody (or fragment thereof) may be conjugated to a therapeutic moiety such as a therapeutic agent or a radioactive metal ion. Therapeutic agents include, but are not limited to, antimetabolites (*e*.*g*., methotrexate, 6-mercaptopurine, 6-thioguanine, cytarabine, 5-fluorouracil decarbazine), alkylating agents (*e*.*g*., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e*.*g*., daunorubicin (formerly daunomycin) and doxorubicin), antibiotics (*e*.*g*., dactinomycin (formerly actinomycin), bleomycin, mithramycin, and anthramycin (AMC), and anti-mitotic agents (*e*.*g*., vincristine and vinblastine).

The conjugates of the invention can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, alpha-interferon, beta-interferom, nerve growth factor, platelet derived growth factor, tissue plasminogen activator; or biological response modifiers such as, for example, lymphokines, interleukin-1 ("IL-1"), interleukin-2 ("IL-2"), interleukin-6 ("IL-6"), granulocyte macrophase colony stimulating factor ("GM-CSF"), granulocyte colony stimulating factor ("G-CSF"), or other growth factors.

Techniques for conjugating such therapeutic moiety to antibodies are well known, see, *e.g.*, Arnon *et al*., "Monoclonal Antibodies For Immunotargeting Of Drugs In Cancer Therapy", in Monoclonal Antibodies And Cancer Therapy, Reisfeld *et al.* (eds.), pp. 243-56 (Alan R. Liss, Inc. 1985); Hellstrom *et al*., "Antibodies For Drug Delivery", in Controlled Drug Delivery (2nd Ed.), Robinson *et al*. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera *et al*. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin *et al.* (eds.), pp. 303-16 (Academic Press. 1985), and Thorpe *et al.,* "The Preparation And Cytotoxic Properties Of Antibody-Toxin Conjugates-, Immunol. Rev., 62:119-58 (1982). Alternatively, an antibody can be conjugated to a second antibody to form an antibody heteroconjugate as described by Segal in U.S. Patent No. 4,676,980.

The nucleic acid molecules used in the methods of the invention can be inserted into vectors and used as gene therapy vectors. Gene therapy vectors can be delivered to a subject by, for example, intravenous injection, local administration (see U.S. Patent 5,328,470) or by stereotactic injection (see, *e*.*g*., Chen *et al*. (1994) *Proc*. *Natl. Acad Sci. USA* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e*.*g*., retroviral vectors, the pharmaceutical preparation can include one or more cells which produce the gene delivery system.

### B. Pharmacogenomics

In conjunction with the therapeutic methods of the invention, pharmacogenomics *(i.e.,* the study of the relationship betweeen a subject's genotype and that subject's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a P-selectin antagonist, *e.g.*, soluble PSGL-1, as well as tailoring the, dosage and/or therapeutic regimen of treatment with an agent which modulates PSGL-1 activity.

Pharmacogenomics deals with clinically significant hereditary variations in the response to drugs due to altered drug disposition and abnormal action in affected persons. See, for example, Eichelbaum, M. *et al.* (1996) *Clin. Exp*. *Pharmacol*. *Physiol.* 23(10-11): 983-985 and Linder, M.W. *et al*. (1997) *Clin. Chem.* 43(2):254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate aminopeptidase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers (*e.g.*, a "bi-allelic" gene marker map which consists of 60,000-100,000 polymorphic or variable sites on the human genome, each of which has two variants). Such a high-resolution genetic map can be compared to a map of the genome of each of a statistically significant number of patients taking part in a Phase II/III drug trial to identify markers associated with a particular observed drug response or side effect. Alternatively, such a high resolution map can be generated from a combination of some ten million known single nucleotide polymorphisms (SNPs) in the human genome. As used herein, a "SNP" is a common alteration that occurs in single nucleotide base in a stretch of DNA. For example, a SNP may occur once per every 1000 bases of DNA. A SNP may be involved in a disease process, however, the vast majority may not be disease-associated. Given a genetic map based on the occurrence of such SNPs, individuals can be grouped into genetic categories depending on a particular pattern of SNPs in their individual.genome. In such a manner, treatment regimens can be tailored to groups of genetically similar individuals, taking into account traits that may be common among such genetically similar individuals.

Alternatively, a method termed the "candidate gene approach "can be utilized to identify genes that predict drug response. According to this method, if a gene that encodes a drug target is known (*e.g.*, a PSGL-1 protein of the present invention), all common variants of that gene can be fairly easily identified in the population and it can be determined if having one version of the gene versus another is associated with a particular drug response.

As an illustrative embodiment, the activity of drug metabolizing enzymes is a major determinant of both the intensity and duration of drug action. The discovery of genetic polymorphisms of drug metabolizing enzymes (*e.g*., N-acetyltransferase 2 (NAT 2) and the cytochrome P450 enzymes CYP2D6 and GYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. The other extreme are the so called-rapid metabolizers who do not respond to standard doses. Recently, the molecular basis of ultra-rapid metabolism has been identified to be due to CYP2D6 gene amplification.

Alternatively, a method termed the "gene expression profiling" can be utilized to identify genes that predict drug response. For example, the gene expression of an animal dosed with a drug (*e*.*g*., a PSGL-1 molecule or P-selectin antagonist of the present invention) can give an indication whether gene pathways related to toxicity have been turned on.

Information generated from more than one of the above pharmacogenomics approaches can be used to determine appropriate dosage and treatment regimens for prophylactic or therapeutic treatment of a subject. This knowledge, when applied to dosing or drug selection, can avoid adverse reactions or therapeutic failure and, thus, enhance therapeutic or prophylactic efficiency when treating or preventing thrombosis with an agent which modulates PSGL-1 activity.

### V. Screening Assays:

The invention provides methods (also referred to herein as "screening assays") for identifying modulators, *i*.*e*., candidate or test compounds or agents (*e*.*g*., peptides, peptidomimetics, small molecules, ribozymes, or PSGL-1 antisense molecules) which bind to PSGL-1 proteins, have a stimulatory or inhibitory effect on PSGL-1 expression or PSGL-1 activity, or have a stimulatory or inhibitory effect on the expression or activity of a PSGL-1 target molecule, *e*.*g*. P-selectin or E-selectin, or have an effect, *e.g.*, inhibition of cellular migration or adhesion, on cells expressing a PSGL-1 target molecule, *e.g*., endothelial cells and activated platelets. Compounds identified using the assays described herein may be useful for modulating thrombosis.

Candidate/test compounds include, for example, 1) peptides such as soluble peptides, including Ig-tailed fusion peptides and members of random peptide libraries (see, e.g., Lam, K.S. et al. (1991) *Nature* 354:82-84; Houghten, R. *et al.* (1991) *Nature* 354:84-86) and combinatorial chemistry-derived molecular libraries made of D- and/or L-configuration amino acids; 2) phosphopeptides (e.g., members of random and partially degenerate, directed phosphopeptide libraries, see, e.g., Songyang, Z. et al. (1993) *Cell* 72:767-778); 3) antibodies (e.g., polyclonal, monoclonal, humanized, anti-idiotypic, chimeric, and single chain antibodies as well as Fab, F(ab')₂, Fab expression library fragments, and epitope-binding fragments of antibodies); and 4) small organic and inorganic molecules (e.g., moledules obtained from combinatorial and natural product libraries).

The test compounds of the present invention can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is limited to peptide libraries, while the other four approaches are applicable to peptide, non-peptide.oligomer or small molecule libraries of compounds (Lam, K.S. (1997) *Anticancer Drug Des.* 12:145).

Examples of methods for the synthesis of molecular libraries can be found in the art, for example in: De Witt *et al.*(1993) *Proc. Natl*. *Acad. Sci. U.S.A.* 90:6909; Erb *et al.* (1994) *Proc. Natl. Acad Sci. USA* 91:11422; Zuckermann *et al.* (1994) *J. Med Chem.* 37:2678; Cho *et al.* (1993) *Science* 261:1303; Carrell *et al.* (1994) *Angrew. Chem. Int*. *Ed*. *Engl.* 33:2059; Carell *et al*. (1994) *Angew*. *Chem*. *Int. Ed. Engl.* 33:2061; and Gallop *et al.* (1994) *J. Med. Chem.* 37:1233.

Libraries of compounds may be presented in solution (*e.g.*, Houghten (1992) *Biotechniques* 13:412-421), or on beads (Lam (1991) *Nature* 354:82-84), chips (Fodor (1993) *Nature 364:555-556),* bacteria (Ladner USP 5,223,409), spores (Ladner USP '409), plasmids *(Cull et al.* (1992) *Proc Natl Acad Sci USA* 89:1865-1869) or phage (Scott and Smith. (1990) *Science* 249:386-390; Devlin (1990) *Science* 249:404-406; Cwirla *et al.* (1990) *Proc. Natl. Acad. Sci.* 87:6378-6382; Felici (1991) *J*. *Mol Biol.* 222:301-310; Ladner *supra*).

Assays that may be used to identify compounds that modulate activity and P-selectin activity include assays for cell adhesion using ⁵¹Cr-labelled cells, *e.g.*, leukocytes (as described in, for example, Kennedy *et al* (2000) *Br J Pharmacology* 130(1):95), and assays for cell migration, *e.g.*, platelet, neutrophil and leukocyte migration (as described in for example Kogaki *et al*. (1999) *Cardiovascular Res* 43(4):968) and Bengtsson *et al*. (1999) *Scand J Clin Lab. Invest* 59(6):439).

In one aspect, an assay is a cell-based assay in which a cell which expresses a PSGL-1 protein or biologically active portion of the PSGL-1 protein that is believed to be involved in the binding of P-selectin (*e.g.*, amino acid residues 42 to 60 of SEQ ID NO:2), or E-selectin, is contacted with a test compound, and the ability of the test compound to modulate PSGL-1 activity is determined. In a preferred embodiment, the biologically active portion of the PSGL-1 protein includes a domain or motif that is capable of interacting with P-selectin or inhibiting P-selectin mediated cellular adhesion. Determining the ability of the test compound to modulate PSGL-1 activity can be accomplished by monitoring, for example, cell adhesion or cell migration. The cell, for example, can be of mammalian origin, *e.g.*, an endothelial cell or a leukocyte.

The ability of the test compound to modulate PSGL-1 binding to a substrate or to bind to PSGL-1 can also be determined. Determining the ability of the test compound to modulate PSGL-1 binding to a substrate can be accomplished, for example, by coupling the PSGL-1 substrate with a radioisotope or enzymatic label such that binding of the PSGL-1 substrate to PSGL-1 can be determined by detecting the labeled PSGL-1 substrate in a complex. Alternatively, PSGL-1 could be coupled with a radioisotope or enzymatic label to monitor the ability of a test compound to modulate PSGL-1 binding to a PSGL-1 substrate in a complex. Determining the ability of the test compound to bind PSGL-1 can be accomplished, for example, by coupling the compound with a radioisotope or enzymatic label such that binding of the compound to PSGL-1 can be determined by detecting the labeled PSGL-1 -compound in a complex. For example, PSGL-1 substrates can be labeled with ¹²⁵I, ³⁵S, ¹⁴C, or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product

It is also within the scope of this invention to determine the ability of a compound to interact with PSGL-1 without the labeling of any of the interactants. For example, a microphysiometer can be used to detect the interaction of a compound with PSGL-1 without the labeling of either the compound or the PSGL-1 (McConnell, H. M. *et al.* (1992) *Science* 257:1906-1912). As used herein, a "microphysiometer" (*e*.*g*., Cytosensor) is an analytical instrument that measures the rate at which a cell acidifies its environment using a light-addressable potentiometric sensor (LAPS). Changes in this acidification rate can be used as an indicator of the interaction between a compound and PSGL-1.

In yet another embodiment, an assay of the present invention is a cell-free assay in which a PSGL-1 protein or biologically active portion thereof (*e.g.*, a fragment of a PSGL-1 protein which is capable of binding P-selectin) is contacted with a test compound and the ability of the test compound to bind to or to modulate (*e*.*g*., stimulate or inhibit) the activity of the PSGL-1 protein or biologically active portion thereof is determined. Preferred biologically active portions of the PSGL-1 proteins to be used in assays of the present invention include fragments which participate in interactions with non-PSGL-1 molecules, *e.g*., fragments with high surface probability scores. Binding of the test compound to the PSGL-1 protein can be determined either directly or indirectly as described above. Determining the ability of the PSGL-1 protein to bind to a test compound can also be accomplished using a technology such as real-time Biomolecular Interaction Analysis (BIA) (Sjolander, S. and Urbaniczky, C. (1991) *Anal*. *Chem*. 63:2338-2345; Szabo *et al.* (1995) *Curr. Opin. Struct. Biol.* 5:699-705). As used herein, "BIA" is a technology for studying biospecific interactions in real time, without labeling any of the interactants (*e.g.*, BIAcore). Changes in the Optical phenomenon of surface plasmon resonance (SPR) can be used as an indication of real-time reactions between biological molecules.

In more than one embodiment of the above assay methods of the present invention, it may be desirable to immobilize either PSGL-1 or P-selectin to facilitate separation of complexed from uncomplexed forms of one or both of the proteins, as well as to accommodate automation of the assay. Binding of a test compound to a PSGL-1 protein, or interaction of a PSGL-1 protein with P-selectin in the presence and absence of a test compound, can be accomplished in any vessel suitable for containing the reactants. Examples of such vessels include microtitre plates, test tubes, and microcentrifuge tubes. In one embodiment, a fusion protein can be provided which adds a domain that allows one or both of the proteins to be bound to a matrix. For example, glutathione-S-transferase/PSGL-1 fusion proteins or glutathione-S-transferase/target fusion proteins can be adsorbed onto glutathione sepharose beads (Sigma Chemical, St Louis, MO) or glutathione derivatized microtitre plates, which are then combined with the test compound or the test compound and either the target protein or PSGL-1 protein, and the mixture incubated under conditions conducive to complex formation (*e*.*g*., at physiological conditions for salt and pH). Following incubation, the beads or microtitre plate wells are washed to remove any unbound components, the matrix is immobilized in the case of beads, and complex formation is determined either directly or indirectly, for example, as described above. Alternatively, the complexes can be dissociated from the matrix, and the level of PSGL-1 binding or activity determined using standard techniques.

Other techniques for immobilizing proteins on matrices can also be used in the screening assays of the invention. For example, either a PSGL-1 protein or a P-selectin molecule can be immobilized utilizing conjugation of biotin and streptavidin. Biotinylated PSGL-1 protein or P-selectin protein can be prepared from biotin-NHS (N-hydroxy-succinimide) using techniques known in the art (*e.g*., biotinylation kit, Pierce Chemicals, Rockford, IL), and immobilized in the wells of streptavidin-coated 96 well plates (Pierce Chemical). Alternatively, antibodies which are reactive with PSGL-1 protein or P-selectin but which do not interfere with binding of the PSGL-1 protein to its target molecule can be derivatized to the wells of the plate, and unbound target or PSGL-1 protein is trapped in the wells by antibody conjugation. Methods for detecting such complexes, in addition to those described above for the GST-immobilized complexes, include immunodetection of complexes using antibodies reactive with the PSGL-1 protein or P-selectin, as well as enzyme-linked assays which rely on detecting an enzymatic activity associated with the PSGL-1 protein or P-selectin.

In yet another aspect of the invention, the PSGL-1 protein or fragments thereof (*e.g.*, a fragment capable of binding P-selectin or E-selectin) can be used as "bait proteins" in a two-hybrid assay or three-hybrid assay (see, *e.g.*, U.S. Patent No. 5,283,317; Zervos *et al.* (1993) Cell 72:223-232; Madura *et al.* (1993) *J*. *Biol. Chem.* 268:12046-12054; Bartel *et al.* (1993) *Biotechniques* 14:920-924; Iwabuchi *et al.* (1993) *Oncogene* 8:1693-1696; and Brent WO94/10300), to identify other proteins, which bind to or interact with PSGL-1 ("PSGL-1-binding proteins" or "PSGL-1-bp) and are involved in PSGL-1 activity. Such PSGL-1-binding proteins are also likely to be involved in the propagation of signals by the PSGL-1 proteins or PSGL-1 targets as, for example, downstream elements of a PSGL-1-mediated signaling pathway. Alternatively, such PSGL-1-binding proteins are likely to be PSGL-1 inihibitors.

The two-hybrid system is based on the modular nature of most transcription factors, which consist of separable DNA-binding and activation domains. Briefly, the assay utilizes two different DNA constructs. In one construct, the gene that codes for a PSGL-1 protein is fused to a gene encoding the DNA binding domain of a known transcription factor (*e.g.*, GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a PSGL-1-dependent complex, the DNA-binding and activation domains of the transcription factor are brought into close proximity. This proximity allows transcription of a reporter gene (*e.g.*, LacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the PSGL-1 protein.

In another aspect, the invention pertains to a combination of two or more of the assays described herein. For example, a modulating agent can be identified using a cell-based or a cell-free assay, and the ability of the agent to modulate the activity of a P-selectin ligand antagonist can be confirmed *in vivo, e.g.,* in an animal model, such as an animal model for thrombosis. Animal models for thrombosis include those described in, at least, for example, Leadley *et al.* (2000) *J Pharmacol Toxicol Methods* 43:101, and Dorffler-Melly, *et al.* (2000) *Basic Res Cardiol* 95:503.

Moreover, a PSGL-1 modulator identified as described herein *(e.g.,* an antisense PSGL-1 nucleic acid molecule, a PSGL-1-specific antibody, or a small molecule) can be used in an animal model to determine the efficacy, toxicity, or side effects of treatment with such a modulator. Alternatively, a PSGL-1 modulator identified as described herein can be used in an animal model to determine the mechanism of action of such a modulator.

This invention is further illustrated by the following examples which should not be construed as limiting. The contents of all references, patents and published patent applications cited throughout this application, as well as the Figures and the Sequence Listing, are incorporated herein by reference.

The following items also illustrate the invention.
1. A method for treating or inhibiting thrombosis in a subject comprising administering a composition comprising an effective amount of a P-selectin antagonist.
2. The method of item 1, wherein the P-selectin antagonist is a soluble PSGL-1 protein or a fragment thereof having P-selectin ligand activity.
3. The method of item 2, wherein the soluble PSGL-1 protein is human PSGL-1.
4. The method of item 2, wherein the soluble PSGL-1 protein is a recombinant protein.
5. The method of item 2, wherein the soluble PSGL-1 protein comprises an r c portion of an immunoglobulin.
6. The method of item 5, wherein the immunoglobulin is human IgG,.
7. The method of item 2, wherein the soluble PSGL-1 protein is a recombinant human PSGL-Ig fusion protein.
8. The method of item 2, wherein the soluble PSGL-1 protein comprises an extracellular domain of human PSGL-1 protein, or a fragment thereof, having P-selectin ligand activity.
9. The method of item 8, wherein the fragment comprises the amino acid sequence set forth in SEQ ID NO : 2 from amino acid 42 to amino acid 60.
10. The method of item 8, wherein the fragment comprises the amino acid sequence set forth in SEQ ID NO: 2 from amino acid 42 to amino acid 88.
11. The method of item 8, wherein the fragment comprises the amino acid sequence set forth in SEQ ID NO: 2 from amino acid 42 to amino acid 118.
12. The method of item 8, wherein the fragment comprises the amino acid sequence set forth in SEQ ID NO: 2 from amino acid 42 to amino acid 189.
13. The method of item 8, wherein the fragment comprises the amino acid sequence set forth in SEQ ID NO: 2 from amino acid 42 to amino acid 310.
14. The method of item 2, wherein the soluble PSGL-1 protein comprises the amino acid sequence from amino acid 42 to amino acid 88 of SEQ ID NO: 2 fused at its C-terminus to an Fc portion of an immunoglobulin.
15. The method of item 8,wherein the soluble PSGL-1 protein further comprises an Fc portion of an immunoglobulin.
16. The method of item 1, wherein the subject is human.
17. The method of item 1, wherein the P-selectin antagonist is administered to the subject prior to thrombus formation.
18. The method of item 2, wherein the effective amount of soluble PSGL-1 protein or fragment thereof is between approximately 0.1 mg/kg and 10 mg/kg.
19. The method of item 18, wherein the effective amount of soluble PSGL-1 protein is approximately 1 mg/kg.
20. The method of item 19, wherein the effective amount of soluble PSGL-1 protein is selected from the group consisting of 0.1 mg/kg, 0.25 mg/kg, 0.5 mg/kg, 0.75 mg/kg, 1.0 mg/kg, 1.25 mg/kg, 1.5 mg/kg, 1.75 mg/kg, 2.0 mg/kg, 2.25 mg/kg, 2.5 mg/kg, 3.0 mg/kg, and 3.5 mg/kg.
21. A method for inhibiting cell adhesion to blood vessels in a subject comprising administering a composition comprising an effective amount of soluble PSGL-1, or a fragment thereof having P-selectin ligand activity.
22. The method of item 21, wherein the cells are selected from the group consisting of leukocytes and platelets.
23. A method for increasing the movement of cells relative to blood vessels in a subject comprising administering a composition comprising an effective amount of soluble PSGL-1, or a fragment thereof having P-selectin ligand activity.
24. The method of item 23, wherein the cells are selected from the group consisting of leukocytes and platelets.
25. A method for inhibiting the effect of a thrombus-inducing agent in a subject comprising administering a composition comprising an effective amount of an effective amount of soluble PSGL-1, or a fragment thereof having P-selectin ligand activity.
26. The method of item 25, wherein the effect of the thrombus inducing agent is on cells selected from the group consisting of leukocytes and platelets.
27. The method of item 25, wherein the thrombus-inducing agent is LTC₄.

### EXAMPLES

### EXAMPLE 1: P-SELECTIN LIGAND PROTEIN FUSION

This example describes the production of a dimeric P-selectin ligand fusion protein (also referred to herein as rPSGL-Ig): A cDNA was constructed encoding the signal peptide, PACE cleavage site and first 47 amino acids of the mature P-selectin. ligand sequence fused to a mutated Fc region of human IgG₁ at His224 of the native Fc sequence. The sequence of the cDNA construct is reported as SEQ ID NO:3. The fusion point is a novel NotI site at nucleotide 261. The amino acid sequence encoded by the cDNA construct is reported as SEQ ID NO:4. The mature amino acid sequence of the encoded fusion protein begins at amino acid 42 of SEQ ID NO:4. The mutations in the Fc portion were a change of Leu234 and Gly237 of the native Fc sequence to Ala.

### EXAMPLE 2: EFFECT OF SOLUSLE P-SELECTIN GLYCOPROTEIN LIGAND-1 IN A MODEL OF DEEP VEIN THROMBOSIS (DVT)

### Materials and Methods

### Experimental Protocol for DVT.

22 male domestic shorthair cats (1.8 kg-3.2kg) were fasted for 18-24 hours and injected intramuscularly (i.m.) with ketamine hydrochloride at a dose of 35 mg/kg. Animals were intubated with a 3-0 gauge endotracheal tube and anesthetized with 1-2 % insoflurance at a flow rate of 1ml/min. Prior to surgery, animals were injected intravenously with either saline (vehicle) (n=7), 4 mg/kg dimPSGL-1 (n=5) or 4 mg/kg tetPSGL-1 (n=5) in 3 ml saline. The necks of the animals were shaved and an incision was made, exposing the underlying jugular vein. The contralateral jugular vein was not manipulated. The jugular vein was gently freed of surrounding connective issue by blunt dissection. Following exposure, jugular veins were occluded with a vascular clamp for 2 hours. Control veins (n=5) were obtained from animals that were not surgically manipulated, following 2 hours of anesthesia. After venous stasis, side branches and the distal end of the vein were tied off with silk suture and veins were perfused with Ca⁺⁺-Mg⁺ free Tyrodes buffer to remove non-adherent blood cells. Subsequently, the vein was reclamped to prevent the re-entry of blood cells. Immediately thereafter, the vein was perfused with 1 % gluteraldehde (in Ca⁺⁺- Mg⁺ free Tyrodes buffer) and tied off under physiological pressure. Veins were harvested, and prepared for scanning electron microscopy (SEM). 30-50 regions of a venous segment were observed and given a histological score of inflammation, as follows:
0-Intact endothelium with no adherent leukocytes and/or platelets
1-Intact endothelium with some adherent leukocytes and/or platelets
2 - Focal endothelial cell damage with adherent leukocytes and/or platelets
3 - Focal endothelial cell damage with surface thrombosis and/or migrating leukocytes
4 - Focal endothelial cell damage with migrating leukocytes, adherent leukocytes and/or platelets and/or fibrin
5 - Extensive endothelial cell damage with migrating leukocytes, adherent leukocytes and/or platelets and/or fibrin

### Intravital Microscopy

Male domestic shorthair cats (n=22, body wt=1.8-3.2 kg) were fasted for 18-24 hours before surgery. The animals were initially sedated with Ketamine hydrochloride (50 mg/kg, i.m.), and subsequently intubated with a 3-0 gauge endotrachel tube. Animals were anesthetized with 1-2% isoflurane at a flowrate of 1 L/min. The animal's body temperature was maintained at 37°C by using a heated circulating water blanket. The right carotid artery and left jugular vein were cannulated with polyethylene tubing (PE-190) for the measurement of systematic arterial pressure (Stratham P10EZ and Grass physiological recorder) and the administration of reagents, respectively. Prior to laparotomy, all animals were administered 20 mg/kg sodium cromolyn intravenously to reduce any effects of mast cell degranulation on baseline leukocyte rolling. The animals were placed in a supine position and a midline abdominal incision was made. A segment of the small intestine was gently exteriorized, placed over an optically clear viewing window and superfused with bicarbonate buffered-saline (BBS) (pH=7.4) at 37°C. All exposed tissues were draped in BBS soaked gauze and covered with saran wrap to prevent evaporation. The mesenteric preparation was observed through an intravital microscope (Zeiss Axioscope FS) with a 40X (NA 0.75) water immersion objective lens and a 10X eye-piece. The image of the post-capillary venule was recorded with a video camera (Panasonic GP-KR222) and a video-cassette recorder (Sony SVT-S3100) for off-line analysis of leukocyte rolling and adhesion.

Single unbranched mesenteric venules (20-45 um diameter) were selected for each study. Venular diameter (Dᵥₑₙ) and red blood cell (RBC) velocity (V_{RBC}) were determined using image shearing and two-slit photometric techniques, respectively. Mean blood velocity (Vₘₑₐₙ) and the Newtonian wall shear rate (γwall) were determined as (V_{RBC}/1-6 and Vₘₑₐₙ/Dᵥₑₙ) x 8 respectively. The number of rolling and adherent leukocytes was determined off-line during playback of videotaped images. Rolling leukocytes were defined as leukocytes that moved at a velocity less than that of RBCs in a given vessel and evaluated using frame-by-frame analysis. The number of rolling leukocytes (flux) were determined by counting all visible cells passing through a perpendicular plane to the vessel axis over a 5 minute period. Leukocyte rolling velocity was assessed by measuring the period of time required for a leukocyte to roll a given distance and averaged for 10 leukocytes per venule. The number of rolling leukocytes present in the venule at any one time was calculated by flux/V_{wbc} and expressed as number of rolling leukocytes per 100 um length of venule. A leukocyte was defined as adherent if it remained stationary to the endothelium for longer than 30 sec. The total number of adherent leukocytes was determined over a 5-minute period.

Prior to laparotomy, animals received either 20 mg/kg cromolyn and either saline (control) (n=12), 1 mg/kg dimPSGL-1 (n=5), 0.5 mg/kg tetPSGL-1 (n=3), or 0.1 mg/kg tretPSGL-1(n=5), intravenously. After exteriorization, the mesentery was allowed to stabilize for 30 minutes. Subsequently, the venule under observation was videotaped for 5 minutes to obtain baseline measurements of leukocyte rolling and adhesion. In addition, baseline measurements of MAP, V_{RBC,} and D_{VEN} were obtained. Immediately thereafter, the mesentery was superfused with BBS containing 500 pM leukotriene C₄ (LTC₄) for 30 minutes and the venule was videotaped for 5 minutes to record changes in leukocyte rolling and adhesion. After the 5-minute recording period, measurements of MAP, V_{RBC}, and D_{VEN} were obtained to assess the effect of LTC₄.

### RESULTS

SEM micrographs were taken from control cats and cats exposed to 2 hours of venous occlusion in the presence of a tetrameric P-selectin antagonist. In control animals, nominal adherence of leukocytes and platelets was observed on venous epithelium and around venous valves. Although some leukocyte adhesion exists, it is evident that the venous epithelium remains intact (61x and 1270x, respectively).

Following 2 hours of venous occlusion in untreated cats, a low magnification view demonstrates a region of adherent leukocytes and platelets. Adhesion of leukocytes and platelets results in the sloughing of endothelial cells (750x). A high magnification view demonstrates leukocyte emigration and endothelial cell injury; resulting in exposure of the basement cell membrane and platelet adhesion (2700x).

Treatment of animals with 4 mg/kg dimPSGL-1 had no effect on leucocyte and platelet adhesion, or endothelial cell injury alter 2 hours of occlusion. Jugular veins from animals treated with 4 mg/kg tetPSGL-1 show the presence of large thrombi at venous valves (30x and 61x, respectively). Jugular veins from animals treated with 4 mg/kg tetPSGL-1 also show leukocyte-platelet aggregates within and around the thrombus (3660x). Jugular veins from animals treated with 4 mg/kg tetPSGL-1 also show large numbers of leukocytes and platelets were adherent to injured epithelium (1810x).

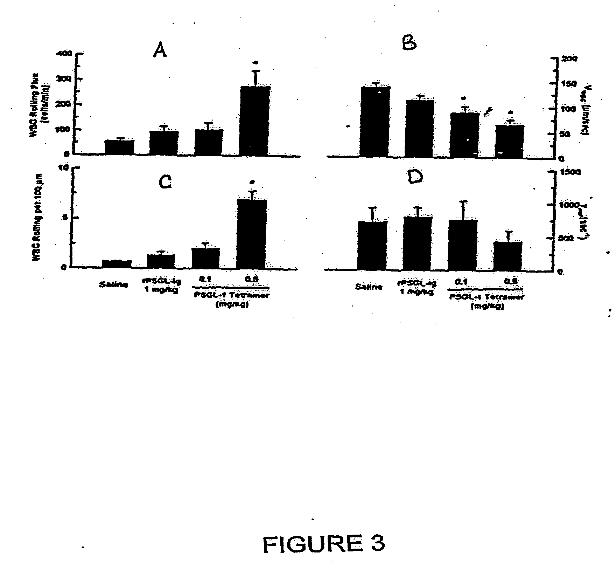

Figure 3 depicts the effect of dimPSGL-1 and tetPSGL-1 on venous wall inflammation following venous occlusion (A) Following 2 hours of occlusion, a significant increase in the level of inflammation was observed, compared to control (p < 0.05) and was invariant with administration of dimSPGL-1. In animals treated with tetPSGL-1, inflammation was observed to be exacerbated, compared to control and 2 hours of occlusion (p<0.05). Large thrombi were found in all animals treatet with 4 mg/kg tetPSLG-1, compared to an absence of thrombi in saline and dimPSGL-1 treated animals. *denotes a value which is significantly different from control p<0.05. # denotes a value which is significantly different from saline treated animals following 2 hours of occlusion, p<0.05. Values shown are mean ± SE.

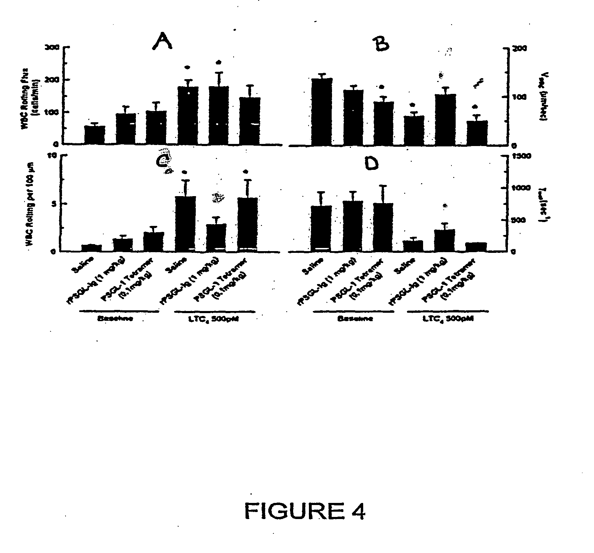

Figure 4 depicts the effect of dimSPGL-1 and tetPSGL-1 on leukocyte rolling under basal conditions. (A) Leukocyte rolling flux was approximately 60 cells/min in saline treated cats and was invariant with administration of dimPSGL-1 and 0.1 mg/kg tetPSFL-1. Rolling flux was significantly elevated in animals receiving 0.5 mg/kg tetPSGL-1, compared to saline-treated animals (p<0.05).(B) While dimPSGL-1 had no effect on baseline leukocyte rolling velocity, 0.1 and 0.5 mg/kg tetPSGL-1 reduced leukocyte rolling velocity by 35 and 50% respectively, compared to saline-treated animals (P<0.05). (C) The changes in leukocyte rolling flux and velocity induced by administration of 0.5 mg/kg tetPSGL-1 resulted in a 7-fold increase in the number of rolling leukocytes per 100 um (p-<0.05) (D) Wall shear rates in venules of saline, dimSPGL-1, and tetPSGL-1 were not significantly different from each other, suggesting that the changes in rolling behavior of leukocytes is not due to alterations in venular hemodynamics. * denotes a value which is significantly different from saline, p <0.05. Values shown are mean ± SE.

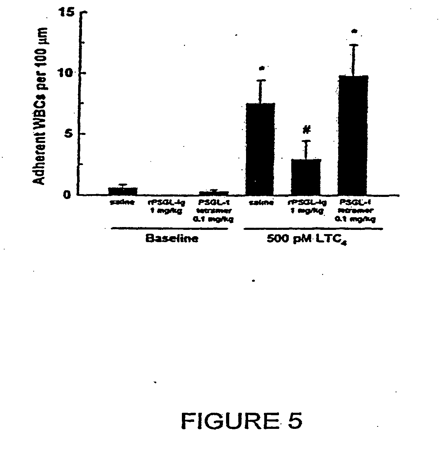

Figure 5 depicts the effect of dimPSGL-1 and tefPSGL-1 on leukocyte rolling following exposure to LTC₄. (A) Following exposure to LTC₄, leukocyte rolling flux was observed to increase 2-3 fold in saline- and dimPSGL-1-treated animals, compared to baseline values (p<0.05). (B) LTC₄ reduced leukocyte rolling velocity in saline- and tetPSGL-1-treated animals by approximately 50% compared to baseline values. However, dimPSGL-1 was effective at abolishing the LTC₄-induced reduction in leukocyte rolling velocity. (C) The changes in leukocyte rolling flux and velocity induced by LTC₄ resulted in an increase in the number of rolling leukocytes per 100 um to approximately 8(P<0.05). The number of rolling leukocytes per 100 um was not significantly elevated in dimPSGL-1-treated animals following exposure LTC₄, and was primarily due to the effect of dimSPGL-1 on leukocyte rolling velocity. (D) Wall shear rates in venules of saline, dimSPGL-1, and tetPSGL-1 were reduced following exposure to LTC₄, however the values of wall shear rate not significantly different between treatment groups. * denotes a value which is significantly different from control, p < 0.05. Values shown are mean ± SE.

Figure 6 depicts the effect of dimPSGL-1 and tetPSGL-1 on leukocyte adhesion following exposure to LTC₄. Leukocyte adhesion was observed to be less than 1 WBC/100 mm under baseline conditions, and was invariant between treatment groups. Following exposure to LTC₄, leukocyte adhesion significantly increased to 8 leukocytes per 100 mm in saline and tetPSGL-1 treated animals. Administration of dimSPGL-1 reduced LTC₄-induced leukocyte adhesion by 60% (p0.05). * denotes a value which is significantly different from control. P <-.05. # denotes a value which is significantly different from LTC₄ P< 0:05. Values shown are mean ± SE.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. Use of a soluble PSGL-1 protein or fragment thereof having P-selectin ligand activity for the preparation of a pharmaceutical composition for treating, inhibiting, or preventing thrombosis in a human subject, wherein the subject has or is at risk of thrombosis due to a disorder, condition, or procedure chosen from:
(a) hypertension, arterial inflammation, rapid ventricular pacing, aortic bending, vascular heart disease, atrial fibrillation, congestive heart failure, sinus node dysfunction, heart failure, atrial flutter, cardiomyopathy, coronary artery disease, coronary artery spasm, arrhythmia;
(b) prolonged sitting, bed rest, immobilization; and
(c) surgical revascularization, coronary artery bypass grafting, rotational atherectomy, stenting, and coronary artery stenting.

2. The use according to claim 1, wherein the subject has the disorder, procedure or condition.

3. The use according to claim 1, wherein the subject is at risk of thrombosis due to the disorder, condition, or procedure.

4. The use according to claim 1, wherein the soluble PSGL-1 protein or fragment thereof having P-selectin ligand activity also comprises a non-P selectin ligand polypeptide.

5. The use according to claim 4, wherein the non-P selectin ligand polypeptide comprises an Fc portion of an immunoglobulin.

6. The use according to claim 5, wherein the immunoglobulin is human IgG1.

7. The use according to claim 1, wherein the soluble PSGL-1 protein is a recombinant human PSGL-Ig fusion protein.

8. The use according to claim 1, wherein the soluble PSGL-1 protein or fragment thereof having P-selectin ligand activity comprises amino acid 42 to amino acid 60 of SEQ ID No: 2.

9. The use according to claim 1, wherein the soluble PSGL-1 protein or fragment thereof having P-selectin ligand activity comprises amino acid 42 to amino acid 88 of SEQ ID No: 2.

10. The use according to claim 1, wherein the soluble PSGL-1 protein or fragment thereof having P-selectin ligand activity comprises amino acid 42 to amino acid 118 of SEQ ID No: 2.

11. The use according to claim 1, wherein the soluble PSGL-1 protein or fragment thereof having P-selectin ligand activity comprises amino acid 42 to amino acid 189 of SEQ ID No: 2.

12. The use according to claim 1, wherein the soluble PSGL-1 protein or fragment thereof having P-selectin ligand activity comprises amino acid 42 to amino acid 310 of SEQ ID No: 2.

13. The use according to claim 1, wherein the soluble PSGL-1 protein comprises the amino acid sequence from amino acid 42 to amino acid 88 of SEQ ID No: 2 fused at its C-terminus to an Fc portion of an immunoglobulin.

14. The use according to claim 13, wherein the immunoglobulin is human IgG 1.

15. The use according to claim 1, wherein the disorder, condition, or procedure is chosen from:
hypertension, arterial inflammation, rapid ventricular pacing, aortic bending, vascular heart disease, atrial fibrillation, congestive heart failure, sinus node dysfunction, heart failure, atrial flutter, cardiomyopathy, coronary artery disease, coronary artery spasm, arrhythmia.

16. The use according to claim 1, wherein the disorder, condition, or procedure is chosen from:
prolonged sitting, bed rest, immobilization.

17. The use according to claim 1, wherein the disorder, condition, or procedure is chosen from:
surgical revascularization, coronary artery bypass grafting, rotational atherectomy, stenting, and coronary artery stenting.
